# EUROPEAN PATENT SPECIFICATION

(11) **EP 3 600 250 B1**
(45) Date of publication and mention of the grant of the patent: **05.10.2022**
(21) Application number: 18772471.1
(22) Date of filing: 20.03.2018
(51) Int. Cl.: A61K 9/00, A61K 9/08, A61K 9/127, A61K 9/14, A61K 9/50, A61K 9/51, A61K 39/00, C12N 5/0783, A61K 31/519, A61K 45/06, A61K 47/69, A61P 35/00, C07K 14/725

(54) **ADOPTIVE TRANSFER OF CAR T CELLS WITH SURFACE-CONJUGATED DRUG-LOADED NANOPARTICLES AND USES THEREOF**
ADOPTIVER TRANSFER VON CAR-T-ZELLEN MIT OBERFLÄCHENKONJUGIERTEN WIRKSTOFFBELADENEN NANOPARTIKELN UND VERWENDUNGEN DAVON
TRANSFERT ADOPTIF DE CELLULES CAR-T À NANOPARTICULES CHARGÉES DE MÉDICAMENT CONJUGUÉES EN SURFACE ET UTILISATIONS ASSOCIÉES

(30) Priority: 20.03.2017 US 201762473594 P
(43) Date of publication of application: 05.02.2020
(73) Proprietor: University of Southern California, Los Angeles, CA 90015 (US)
(72) Inventor: WANG, Pin, Los Angeles, California 90024 (US); KIM, Yu Jeong, Los Angeles, California 90036 (US); SIRIWON, Natnaree, Los Angeles, California 90005 (US)
(74) Representative: Hutter, Anton
(86) International application number: PCT/US2018/023404
(87) International publication number: WO 2018/175473

(56) References cited:
- WO-A1-2016/176558
- WO-A1-2016/176558
- WO-A1-2017/023787
- WO-A2-2015/142675
- US-A1- 2013 071 326
- US-A1- 2014 356 414
- US-A1- 2016 089 452
- US-A1- 2016 256 386
- PAUL A. BEAVIS ET AL: "Targeting the adenosine 2A receptor enhances chimeric antigen receptor T cell efficacy", THE JOURNAL OF CLINICAL INVESTIGATION, vol. 127, no. 3, 6 February 2017 (2017-02-06), pages 929-941, XP055459266, GB ISSN: 0021-9738, DOI: 10.1172/JCI89455
- YOUNG ARABELLA ET AL: "Co-inhibition of CD73 and A2AR Adenosine Signaling Improves Anti-tumor Immune Responses", CANCER CELL, CELL PRESS, US, vol. 30, no. 3, 12 September 2016 (2016-09-12), pages 391-403, XP029725575, ISSN: 1535-6108, DOI: 10.1016/J.CCELL.2016.06.025
- MAXIME AYER ET AL: "Cell-mediated delivery of synthetic nano- and microparticles", JOURNAL OF CONTROLLED RELEASE, vol. 259, 8 February 2017 (2017-02-08), pages 92-104, XP055754344, AMSTERDAM, NL ISSN: 0168-3659, DOI: 10.1016/j.jconrel.2017.01.048
- ELIZABETH L. SIEGLER ET AL: "Combination Cancer Therapy Using Chimeric Antigen Receptor-Engineered Natural Killer Cells as Drug Carriers", MOLECULAR THERAPY, vol. 25, no. 12, 1 December 2017 (2017-12-01), pages 2607-2619, XP055753375, US ISSN: 1525-0016, DOI: 10.1016/j.ymthe.2017.08.010
- NATNAREE SIRIWON ET AL: "CAR-T Cells Surface-Engineered with Drug-Encapsulated Nanoparticles Can Ameliorate Intratumoral T-cell Hypofunction", CANCER IMMUNOLOGY RESEARCH, vol. 6, no. 7, 2 May 2018 (2018-05-02), pages 812-824, XP055753382, US ISSN: 2326-6066, DOI: 10.1158/2326-6066.CIR-17-0502
- BEAVIS et al.: "Targeting the adenosine 2A receptor enhances chimeric antigen receptor T cell efficacy", The Journal of Clinical Investigation, vol. 127, no. 3, 6 February 2017 (2017-02-06), pages 929-941, XP055459266,
- HAN et al.: "Masked Chimeric Antigen Receptor for Tumor-Specific Activation", Molecular Therapy, vol. 25, no. 1, 4 January 2017 (2017-01-04), pages 274-284, XP009195209,

## Description

### FIELD OF THE INVENTION

Various embodiments of the invention provide compositions and methods for treating cancer.

### BACKGROUND OF THE INVENTION

Adoptive CAR-engineered T cell therapy has demonstrated success in treating blood-born tumors, prominently CD19 CARs in leukemia and B cell lymphoma in preclinical and clinical trials. Despite these promising results, the clinical application of CAR-T cell therapy towards solid tumors is limited. These shortcoming is due to the tumor microenvironment that have unique barriers that are absent in hematological malignancies.

Unlike hematological cancers, which circulate throughout the body in the blood stream, solid tumors have their own complex tumor microenvironment (TME), which provides a unique barrier to immunotherapy. To be effective, immune cells must efficiently infiltrate the solid tumor mass and have extended persistence of *in vivo* expanded cells. The TME contains a variety of pro-tumorigenic factors that work to both prevent cancer-killing immune cells from entering the tumor area and dampen the activation of tumor infiltrating lymphocytes (TILs). Many of these immune suppressive mechanisms can also negatively impact adoptively transferred CAR-engineered T cells.

In fact, several studies presented evidence showing the rapid loss of effector functions that limited therapeutic efficacy of CART cells by the strong immunosuppressive environment after being injected into immune-deficient mice bearing established human solid tumors. This tumor induced hypofunction of tumor infiltrated T cells (TILs) is caused by multiple mechanisms.

One of the underlying factors that is responsible for a progressive loss of CAR T-cell effector function in tumor microenvironment is the extracellular inhibitory pathway that gets triggered by abnormally increased concentration of an extracellular immunosuppressive molecule.

A2a adenosine receptor (A2AR) is expressed on the surface of activated T cells. The A2aR pathway is triggered by abnormally high concentrations of the extracellular immunosuppressive molecule adenosine, which has been reported to suppress T cell proliferation and IFN-γ secretion. In the TME, extracellular adenosine triphosphate (ATP) is released in response to tissue damage and cellular stress. ATP in the extracellular environment is converted into adenosine by ectonucleases CD39 and CD73, which are upregulated in the hypoxic TME. Overexpression of CD73 has been observed in multiple aggressive cancers, conferring resistance to antitumor agents. Binding of adenosine to A2aR leads to increased intracellular cyclic AMP (cAMP) production in the TILs. Elevation of intracellular cAMP induces activation of protein kinase A (PKA) and phosphorylation of the cAMP response element binding protein (CREB), which, in turn, abrogates T cell receptor (TCR) signaling and IFN-γ production by reducing the activity of the Akt pathway and inhibiting NF-kB-mediated immune activation.

Studies have demonstrated that the pathway blockade of A2AR by either pharmacological inhibition or genetic deletion significantly improved antitumor immunity by enhancing cytotoxic T cell efficacy and *in* vivo persistence. Beavis et al. report that blockade with SCH58261 increases the anti-tumor effect of CAR T cells when administered together with an anti-PD1 (J Clin Invest. 2017;127(3):929-941). SCH-58261 is a potent and selective antagonist for the adenosine receptor A2A, with more than 50 times higher selectivity for A2A over other adenosine receptors. Despite its therapeutic potential, its clinical development has been hindered by the drug's poor solubility and *in vivo* PK profiles. Furthermore, these small-molecule drugs that act directly on the transferred CAR T cells need to be maintained at high and sustained systemic levels for efficacy. It remains challenging to develop an effective carrier capable of regulating drug circulation time *in vivo* and specifically and efficiently delivering the drug in tumors while minimizing "on-target but off tumor" side effects.

Recently, important advances have been made to employ nanotechnology for drug delivery, enhancing the therapeutic efficacy of several anticancer drugs. Compared to free drugs, drug-loaded nanoparticles can successfully provide targeted delivery with better efficacy and fewer side-effects by prolonging blood circulation time, controlling sustained drug release, reducing systemic toxicities, and increasing drug concentration in cancer tissue through the enhanced permeability and retention (EPR) effect.

However, the EPR effect is highly dependent on adequate vascularization of tumors. Vascularization may be completely lacking in some tumors that exhibit poor blood supply and hypoxia. Additionally, high interstitial fluid pressures within the tumor can act to transport therapeutics back into the bloodstream. Most administered nanoparticles (up to 95%) are reported to accumulate in organs other than tumor, including, for example, liver, spleen and lungs. Hence, efficient delivery and distribution of nanoparticles within the tumor mass remains challenging.

Numerous studies have shown that the addition of targeting moieties on nanoparticles can significantly improve their tumor specificity and accumulation, but these targeting strategies still rely on passive distribution of the nanoparticles through the bloodstream.

Therefore, it is an objective of the present invention to provide a composition and/or a delivery system that infiltrate and persist in tumor mass to inhibit or control the growth of, and reduce tumor size or related symptoms.

It is another objective of the present invention to provide a method of treating a subject with or subject to developing tumor by promoting or rescuing CAR T-cell effector function in the tumor microenvironment.

### SUMMARY OF THE INVENTION

An engineered cell is provided to enhance the efficacy of adoptive CAR T therapy and others for cancer therapy, where active agent-loaded nano- or microparticles are chemically conjugated to the surface of the cell. In various embodiments, the cell to be surface engineered (or modified) are immune effector cells having polynucleotide encoding chimeric antigen receptors (CARs) or having expressed on the surface CARs. Generally, the surface conjugation with particles does not alter the viability, the cytokine secretion function, the cytotoxicity towards tumor cells, or the chemotaxis-driven migration of native CAR T cells that do not have conjugated nanoparticles.

Generally, CAR T cells that are surface conjugated with drug-loaded nanoparticles migrate and retain deep in tumor, e.g., in the suppressive tumor microenvironment; and release the drug for a controlled or sustained period of time therein. Preferably, the drug is a therapeutic or prophylactic agent that can reduce the likelihood or reverse the inhibition on T cells from the suppressive tumor microenvironment.

Exemplary nanoparticles for conjugation include liposomes, such as cross-linked multilamellar liposomes, and controlled release polymeric nanoparticles. Depending on the solubility of the incorporated active agent, hydrophobic polymers or block copolymers may be selected, e.g., poly(lactic acid), poly(glycolic acid) or copolymer thereof, to form nanoparticles for controlled released of active agent therefrom. Generally, nanoparticles are conjugated to each cell at a ratio that does not alter the function of the cell, yet high enough to deliver a high load of active agent per cell. For example, the number of conjugated nanoparticles per cell is between 400 and 350, between 350 and 300, between 300 and 250, between 250 and 200, between 200 and 150, or between 150 and 100. An exemplary conjugation is between maleimide-functionalized particles and cells with surface thiol groups.

Generally, the active agent can be any of small molecule compounds, cytokines, antibodies or antigen-binding fragment thereof. Exemplary active agent for encapsulation, dispersion or otherwise incorporation in the nanoparticles and controlled release from the nanoparticles from the CAR T cell surface include inhibitors and antagonists that alter the tumor microenvironment. In some embodiments, the active agent is an antagonist of adenosine receptor A2a, for example SCH-58261, caffeine and ZM241385. In other embodiments, the active agent may be an antagonist or inhibitor of vascular endothelial growth factor (VEGF) or an antagonist or inhibitor of VEGF receptor (VEGFR).

Exemplary cell types which nano- or microparticles are conjugated to include "chaperone" cells such as natural killer cells, and "therapeutic cells" such as tumor-specific T lymphocytes and hematopoietic stem cells.

In various embodiments, carrier cells are surface conjugated with nanoparticles that deliver active agents that prevent, reverse, or block the inhibition of endogenous T cell function in the microenvironment of aggressive cancer that produces adenosine.

In some embodiments, cross linked multilamellar liposomes (cMLVs), incorporating an A2aR-specific antagonist SCH-58261, are chemically conjugated to the surfaces of CAR T-cells. First, we encapsulated a potent and selective antagonist for the adenosine receptor A2A, SCH, into our cross-linked multilamellar liposomal vesicle (cMLV) drug delivery system. Then, we demonstrated that CAR T cells can be covalently conjugated to our nanoparticle delivery system carrying SCH without affecting their viability or function. To test the therapeutic efficacy of actively targeting delivery system on T cell function in vivo, we developed two preclinical models using a human solid tumor xenograft mouse model that allowed demonstrating two conditions: (1) Prevention of, and (2) Recovery of tumor- induced hypofunction of CAR T cells.

Through extensive *in vivo* and *ex vivo* experimental investigations in both preclinical models, surface engineered CAR T cells with conjugated cMLV that delivers SCH-58261 retain their capacity to migrate into and actively direct drug-loaded cMLVs into tumor sites. Furthermore, CAR T-cMLV(SCH) treatment had the highest anti-tumor efficacy with significantly higher retention of CD3⁺ TILs and more secretion of inflammatory cytokines, such as IFNγ *in vivo,* suggesting that conjugation of SCH-loaded cMLVs directly to CAR T cells markedly increased their therapeutic impact.

In some embodiments, the compositions are co-administered with one or more of immune check point inhibitors, immune modulating agents, or chemotherapeutic agents, simultaneously or sequentially.

### BRIEF DESCRIPTION OF THE DRAWINGS

Exemplary embodiments are illustrated in referenced figures. It is intended that the embodiments and figures disclosed herein are to be considered illustrative rather than restrictive.

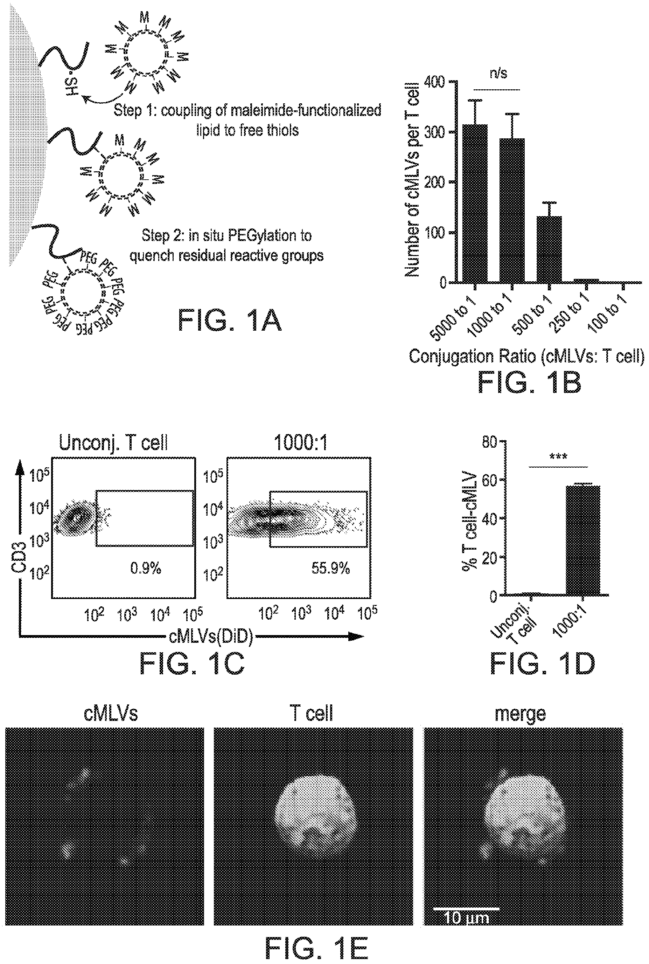

**Figure 1A** depicts in accordance with various embodiments of the invention, a schematic of maleimide-based stable conjugation of nanoparticles (cMLVs) to a CAR T cell. **Figure 1B** depicts a bar graph showing the number of conjugated nanoparticles per T cell at different conjugation ratios (5000 to 1, 1000 to 1, 500 to 1, 250 to 1 and 100 to 1, nanoparticle to T cell ratio). Data represents the mean ± SD of two independent experiments conducted in triplicate. **Figures 1C and 1D** depict the flow cytometry analysis **(1C**) and the corresponding bar graph **(ID)** showing the percentage of cMLV conjugated CD3⁺ T cells (at a conjugation ration of cMLV:T cell = 1000:1). **Figure 1E** shows the single-cell confocal microscopy images of a CAR T cell conjugated with DiD-loaded cMLV in a red channel, a green channel, and a merge image. These CAR T cells were labeled with 1 µM CFSE and washed with PBS prior to conjugation to cMLV(DiD). Confocal microscopy was used to visualize the cMLVs on the CAR T cell surface. Scale bar represents 10 µm. (Red: DiD labeled cMLVs, Green: CFSE labeled CAR T cell) (n = 3, mean ± SD; ns, not significant; ^{∗}p < 0.05; ^{∗∗}p < 0.01; ^{∗∗∗}p < 0.001).

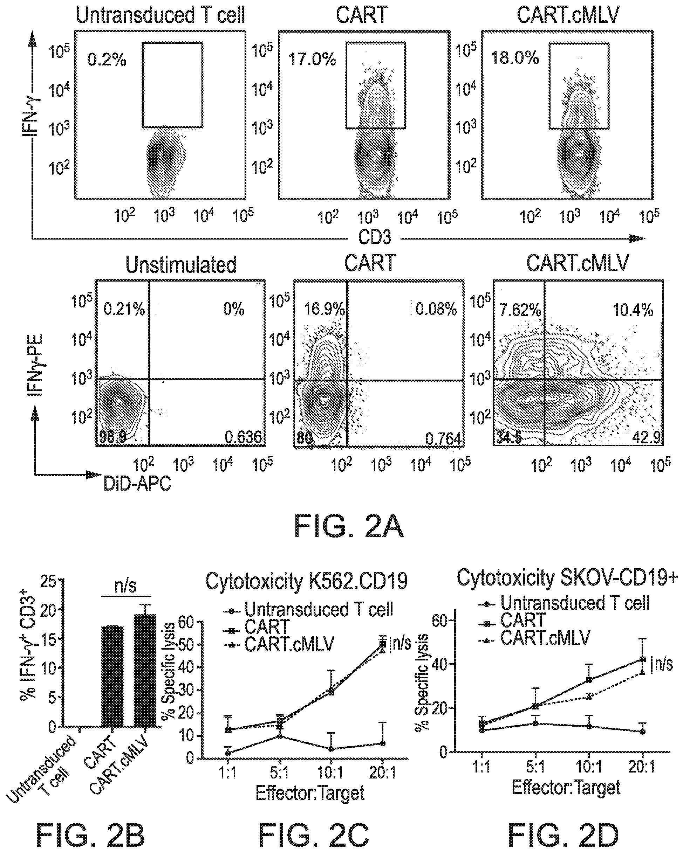

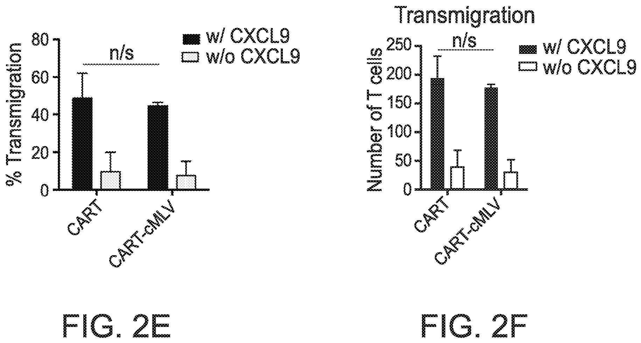

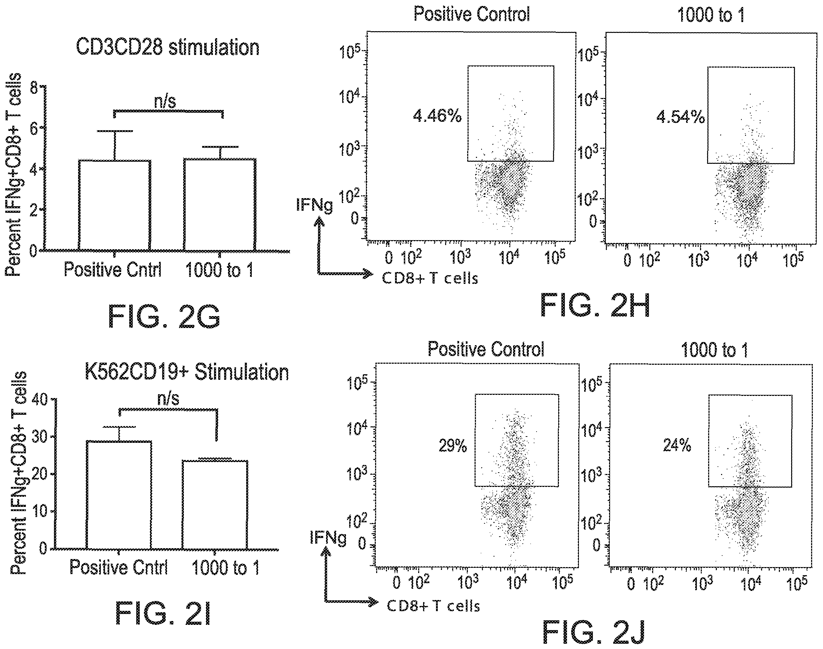

**Figures 2A-2J** depict in accordance with various embodiments of the invention that conjugation of cMLV did not alter CAR T cell function. **Figure 2A and 2B** depict the flow cytometry analysis (2A) and the corresponding bar graph representation **(2B)** from IFNγ staining assays. Representative FACS analysis of CAR-T cells either unconjugated (CART) or CART cells conjugated with empty cMLVs (CART.cMLV) stimulated with SKOV3.CD19 cells for 6 hours to detect IFN-γ release. Untransduced CAR- T cells served as a negative control.IFN-γ was measured with intracellular staining. Figure 1A in lower row show IFNγ secretion of CD3⁺ T cells, either conjugated or unconjugated with T cells. **Figure 2C** depicts nanoparticle conjugation does not affect CAR T cell cytotoxicity against K562-CD19⁺ target tumor. **Figure 2D** depicts nanoparticle conjugation does not affect CAR T cell cytotoxicity against SKOV3-CD19⁺ tumor. Mean % Cytotoxicity ± SD of experiment performed in triplicates. **Figures 2E** and **2F** depict the percentage **(2E)** and the number of transmigrated T cells **(2F)** in a transmigration assay. Either unconjugated (CART) or cMLVs conjugated CAR T cells (CART.cMLV) were seeded in the upper chambers of a Transwell with or without addition of chemoattractant CXCL9 to the lower chambers. After 6 hours of incubation, media from the lower chambers was collected and CAR-T cells were counted. Summarized statistics are displayed in the graphs (n = 3, mean ± SD; ns, not significant; ^{∗}p < 0.05; ^{∗∗}p < 0.01; ^{∗∗∗}p <0.001) All data are representative of at least three independent experiments. **Figures 2G and 2H** depict that the conjugation of cMLV did not alter cytokine secretion by the T cells upon restimulation with anti-human CD3/CD28 antibodies (2G showing a bar graph; 2H showing corresponding flow cytometry spectra). Figures **2I** and 2J depict that the conjugation of cMLV did not alter cytokine secretion by the T cells upon restimulation with K562-CD19⁺ tumor cells (21 showing a bar graph; 2J showing corresponding flow cytometry spectra). Average % ± SD of experiment performed in triplicates.

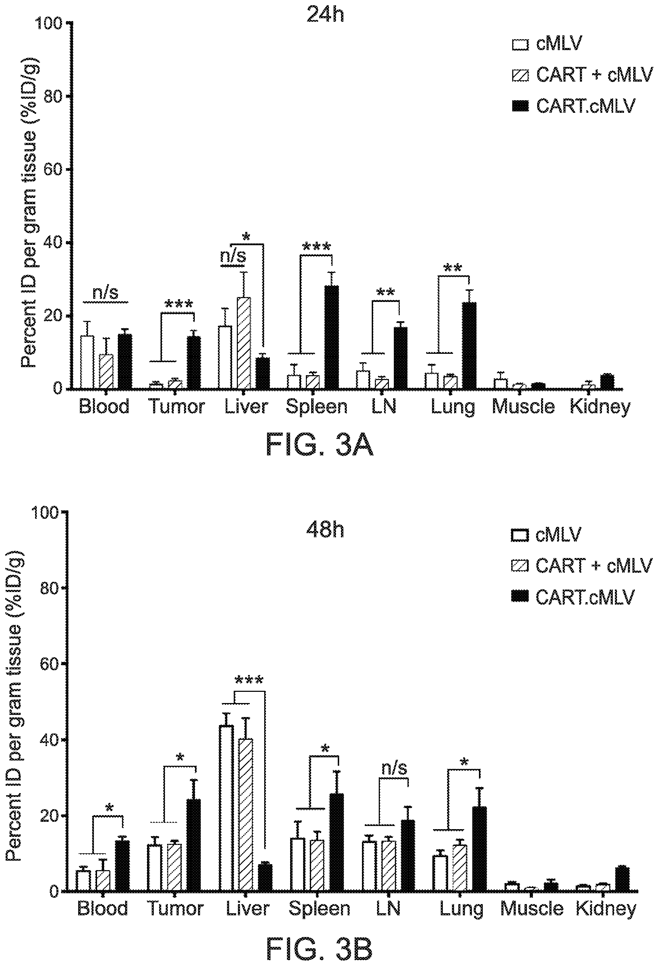

Figures 3A and 3B depict cMLVs bound to CAR T cells show more efficient infiltration to antigen-expressing tumors than free cMLVs. Group of 3 NOD/scid/IL2rγ-/- (NSG) mice bearing subcutaneous SKOV3.CD19 tumors were intravenously injected with 1 × 10⁷ CAR-T cells conjugated (CART.cMLV), co-infused with DiD-labeled cMLVs (CART+cMLV) or an equivalent number of DiD-labeled cMLVs alone (cMLV). After 24 hours **(3A)** and 48 hours **(3B)**, indicated tissues were removed, weighed, and macerated with scissors. Specific DiD tissue fluorescence for each organ was quantified using the IVIS spectrum imaging system and calculated the mean percentage of injected dose per gram of tissue (% ID/g) as final readout. Data shown are pooled from two independent experiments. (n = 3, mean ± SD; ns, not significant; ^{∗}p < 0.05; ^{∗∗}p < 0.01; ^{∗∗∗}p < 0.001).

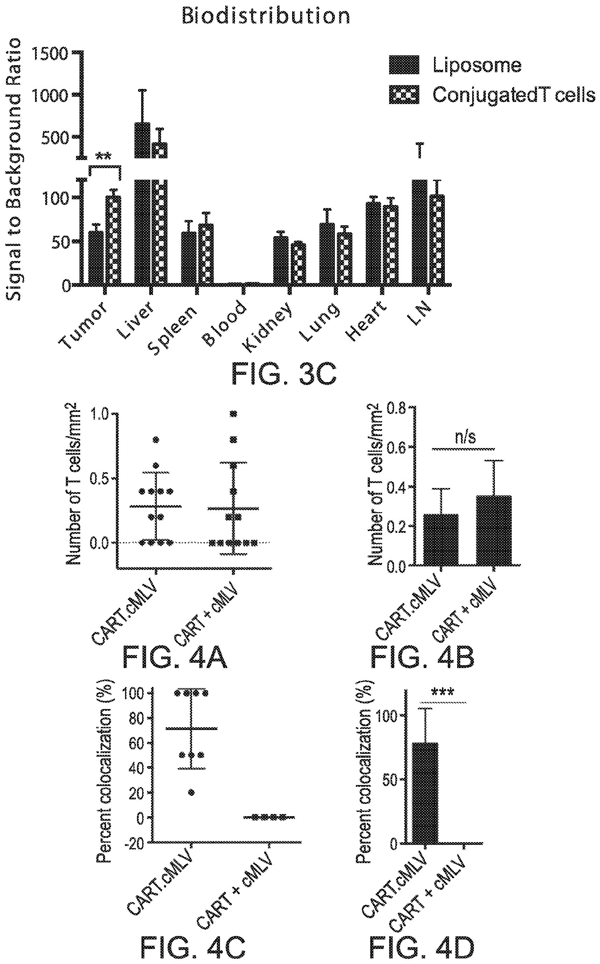

**Figure 3C** depicts nanoparticles conjugated to T cells demonstrated higher accumulation in the tumor compared to free cMLVs. cMLVs labeled with DiD dye were detected with an optical fluorescent microscope. The fluorescence intensity was normalized with organs collected from PBS treated mice. n =3. ^{∗∗}, p<0.01, analyzed by one-way analysis of variance.
**Figures 4A-4D** show the quantifications from confocal microscopic imaging of the co-localization of cMLVs (surface conjugated on CAR T cells) with CAR T cells inside the tumor mass 48 hours post CAR T cell infusion. **Figures 4A and 4B** show the dot plot (4A) and the bar graph (413) of the density of SKOV3.CD19 tumor-infiltrating CAR T cells from either CART.cMLV or CART+cMLVs group. **Figure 4C and 4D** show the dot plot (4C) and the bar graph (4D) of the percentage of tumor infiltrated CAR T cell that are colocalized with cMLVs inside the tumor tissues, (n = 6, mean ± SD; NS, not significant; ^{∗}p < 0.05; ^{∗∗}p <0.01; ^{∗∗∗}p < 0.001)

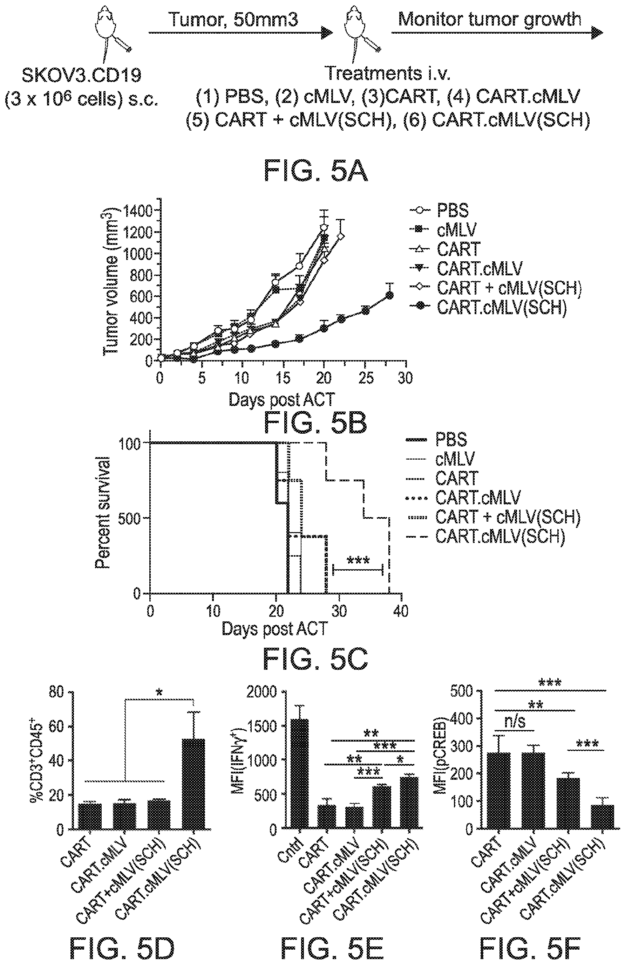

**Figures 5A-5F** depict Anti-CD19 CAR T cells conjugated with SCH58261-releasing cMLVs were prevented from developing hypofunction in animals with SKOV3.CD19 tumors. SKOV3.CD19 cells were injected subcutaneously into the right flank of NSG mice. Mice were randomized into six groups and treated with indicated treatments via i.v. injections. **Figure 5A** is a schematic of targeted *in vivo* delivery of different treatments. **Figure 5B** depicts Tumor size progression as measured with a digital caliper (n = 8, mean ± SD; n/s, not significant; ^{∗}p < 0.05; ^{∗∗}p < 0.01; ^{∗∗∗}p < 0.001). **Figure 5C** depicts the mouse survival curve as calculated using the Kaplan-Meier method. After indicated treatments, flank tumors were harvested and digested for ex vivo analyses. The quantity and function of tumor infiltrated CAR-T cells were evaluated. **Figure 5D** depicts the percentage of CD3⁺CD45⁺ T cells in the tumor at 48 hours post treatment. **Figure 5E** depicts *ex vivo* IFNγ secretion of tumor-infiltrated T cells upon stimulation with antihCD3 and anti-hCD28, 2 days post treatments. **Figure 5F** depicts the detection of phosphorylated CREB expression levels in tumor-infiltrated T cells 2 days post treatments, (n = 3, mean ± SD; n/s, not significant; ^{∗}p < 0.05; ^{∗∗}p < 0.01; ^{∗∗∗}p <0.001).

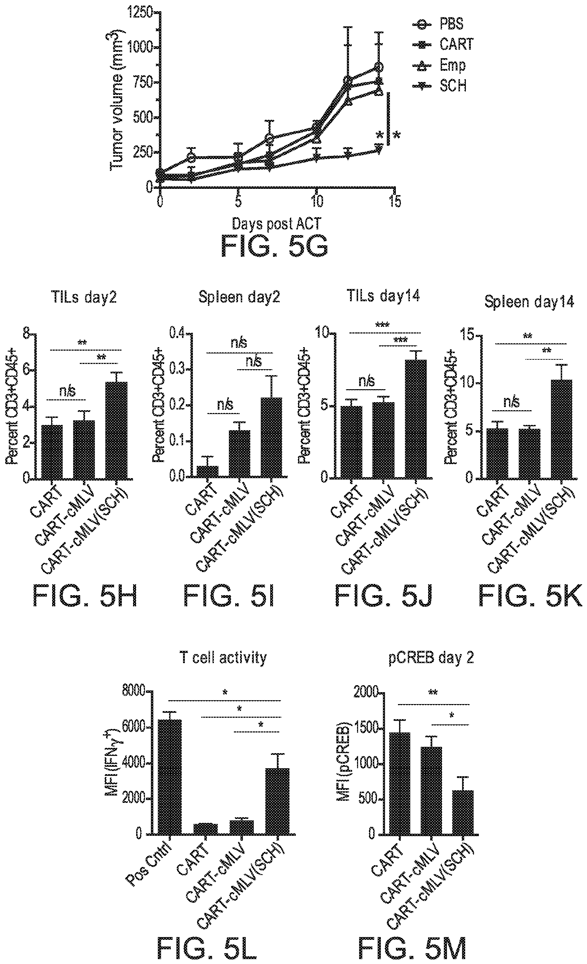

**Figure 5G** depicts CART cell conjugated with cMLV encapsulating SCH58261 demonstrated robust control of tumor growth after adoptive T cell transfer (ACT). Mice bearing established subcutaneous SKOV3-CD19⁺ tumor (100-150mm³) received one of PBS, CART, CART-Emp (denoted as "Emp") or CART-SCH (denoted as "SCH" in the drawing) infusion on day 0. Tumor volume was measured with a vernier caliper and calculated according to the formula (w² x 1) / 2. Data represents mean volume ± SD of an in vivo experiment conducted with n = 15. ^{∗}, p<0.05. ^{∗∗}, p<0.01, analyzed by one-way analysis of variance.
**Figures 5H-5K** depict CART cell conjugated with cMLVs encapsulating SCH58261 demonstrated increased CD3CD45⁺ T cell infiltration and proliferation in both the tumor and the spleen. **Figure 5H** shows TIL analysis on day 2 post ACT. **Figure 5I** shows spleenocyte analysis on day 2 post ACT. **Figure 5J** shows TIL analysis on day 14 post ACT. **Figure 5K** shows spleenocyte analysis on day 14 post ACT. Data represents mean ± SD, n = 5. ^{∗}, p<0.05. ^{∗∗}, p<0.01 and ^{∗∗∗}, p<0.005 analyzed by one-way analysis of variance.
**Figures 5L and 5M** depict CART cell conjugated with cMLVs encapsulating SCH58261 demonstrated increased cytokine secretion and decreased CREB phosphorylation. Figure 5L shows intracellular IFNγ secretion on day 2 post ACT. Figure 5M shows phosphorylated CREB on day 2 post ACT Data represents MFI ± SD, n = 3. ^{∗}, p<0.05. ^{∗∗} p<0.01 analyzed by one-way analysis of variance.

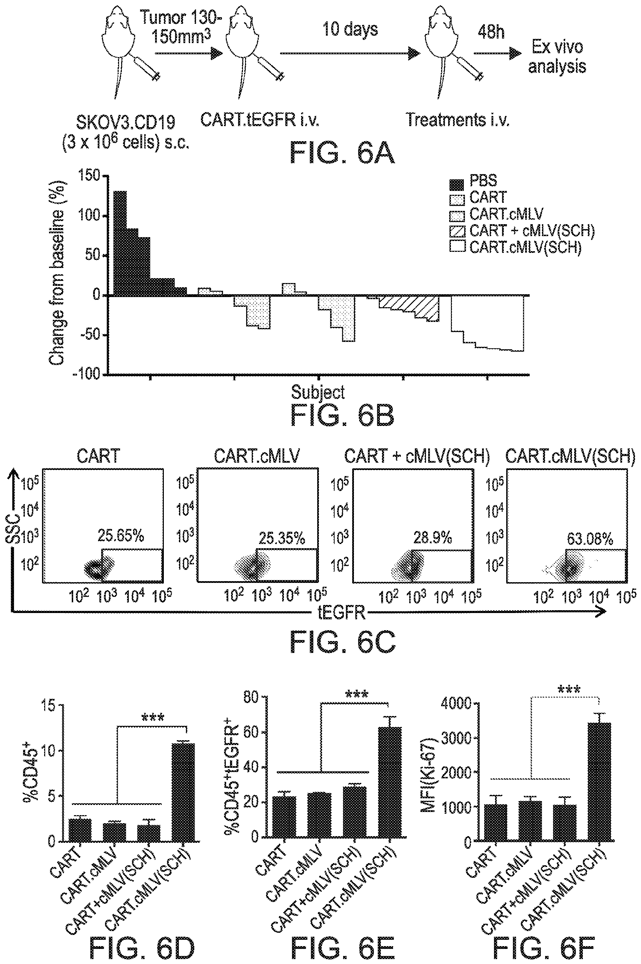

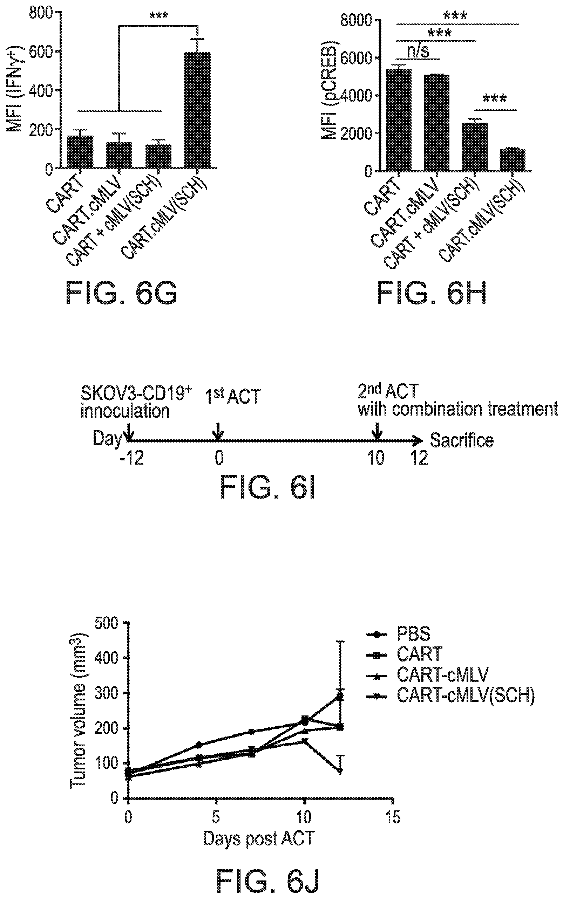

**Figures 6A-6H** depict anti-CD19 CAR T cells conjugated with SCH58261-releasing cMLVs were able to rescue hypofunctional tumor infiltrated T cells in SKOV3.CD19 tumors. **Figure 6A** is a schematic illustration of targeted *in vivo* delivery of CAR T cells conjugated with SCH-releasing cMLVs to inhibit SKOV3.CD19 tumors by rescuing tumor infiltrated CART.tEGFR cells. **Figure 6B** is a waterfall plot displaying the percent change in the tumor size from baseline at Day 35 post i.v. injections, (n = 6, mean ± SD; ns, not significant; ^{∗}p < 0.05; ^{∗∗}p < 0.01; ^{∗∗∗}p < 0.001). **Figure 6C** are representative FACS plots of the percentage of CART.tEGFR cells in the tumor 2 days post indicated treatment. **Figure 6D** depicts the percentage of CD45+ T cells in the tumor 2 days post indicated treatments. **Figure 6E** is a quantitative graph showing the percentage of C ART. tEGFR. cells in the tumor 2 days post indicated treatments. **Figure 6F** shows the detection of Ki-67 expression in C ART. tEGFR. cells 2 days post indicated treatments. **Figure 6G** depicts *ex vivo* IFNy secretion of tumor infiltrated C ART. tEGFR. cells upon stimulation with anti-hCD3 and antihCD28, 2 days post indicated treatments. IFN-γ release was measured with intracellular staining. **Figure 6H** depicts detection of phosphorylated CREB expression levels in C ART. tEGFR. cells 2 days post indicated treatments. (n = 3, mean ± SD; ns, not significant; ^{∗}p < 0.05; ^{∗∗}p < 0.01; ^{∗∗∗}p < 0.001). All data are representative of at least two independent experiments.
Figures 6I and **6J** depict CART cell conjugated with cMLV encapsulating SCH58261 decreased the tumor size after the *rescue* treatment with a second adoptive T cell transfer (ACT) on day 10. **Figure 6I** shows a schematic of the experimental timeline. **Figure 6J** shows on day 10 post initial CART infusion (day 0), mice bearing established subcutaneous SKOV3-CD19⁺ tumor (∼200mm³) received PBS, CART, CART-Emp or CART-SCH infusion. Tumor volume was measured with a vernier caliper and calculated according to the formula (w² x 1) / 2. Data represents mean volume ± SD of an in vivo experiment conducted with n = 15. ^{∗}, p<0.05. ^{∗∗}, p<0.01, analyzed by one-way analysis of variance.

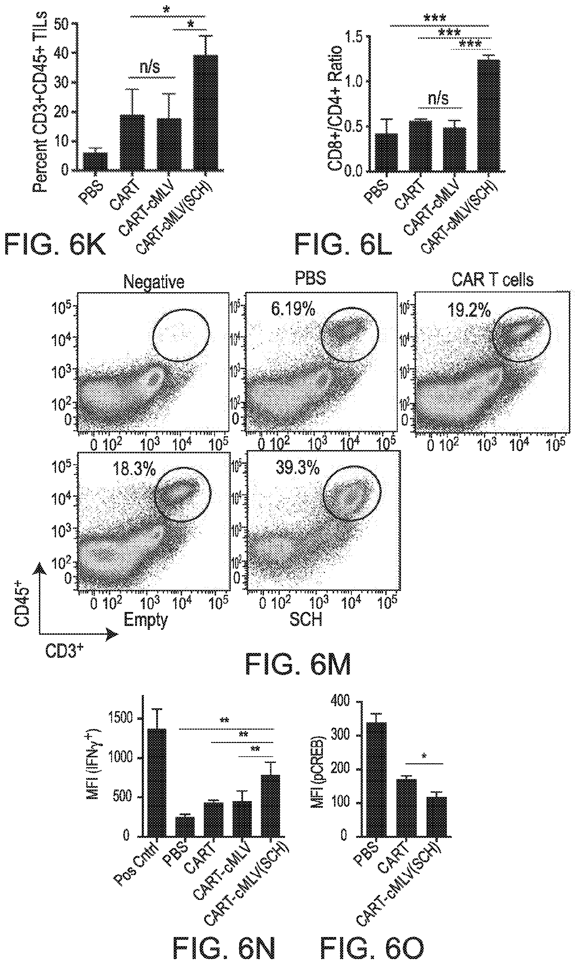

**Figures 6K-6M** depict CART cell conjugated with cMLV encapsulating SCH58261 increased T cell infiltration and proliferation in the tumor. Figure 6K shows the percent CD3CD45⁺ TIL population 48h after second adoptive T cell transfer. Figure 6M shows the pseudo-color plot of CD3CD45⁺ TIL population 48h after second adoptive T cell transfer. Figure 6L shows C])8⁺/CD4⁺T cell ratio. Data represents mean volume ± SD of an in vivo experiment conducted with n = 5. ^{∗}, p<0.05. ^{∗∗}, p<0.01 and ^{***}, p<0.005 analyzed by one-way analysis of variance.
**Figures 6N and 6O** depict CART cell conjugated with cMLVs encapsulating SCH58261 rescued cytokine secretion and decreased CREB phosphorylation of tumor residing T cells. **Figure** 6N shows intracellular IFNγ secretion 48h post treatment. **Figure** 6O shows the phosphorylated CREB 48h post treatment. Data represents MFI ± SD, n = 3. ^{∗}, p<0.05. ^{∗∗}, p<0.01 analyzed by one-way analysis of variance.

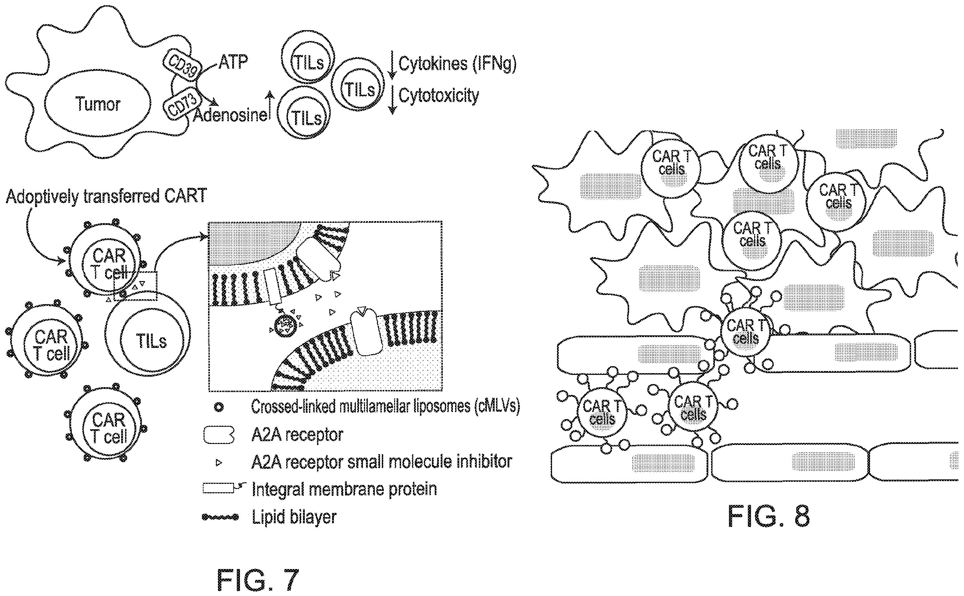

**Figure 7** is a schematic diagram of adoptively transferred cMLV-conjugated CAR T cells in the presence of recipient cells *in vivo.* Maleimide functionalized cMLVs loaded with A2AR small molecule inhibitors are conjugated to CAR-T cells via cell surface thiols.
**Figure 8** depicts in accordance with various embodiments of the invention, schematic diagram of the "rescue" treatment: chaperone CART cell conjugated with drug encapsulating cMLVs.

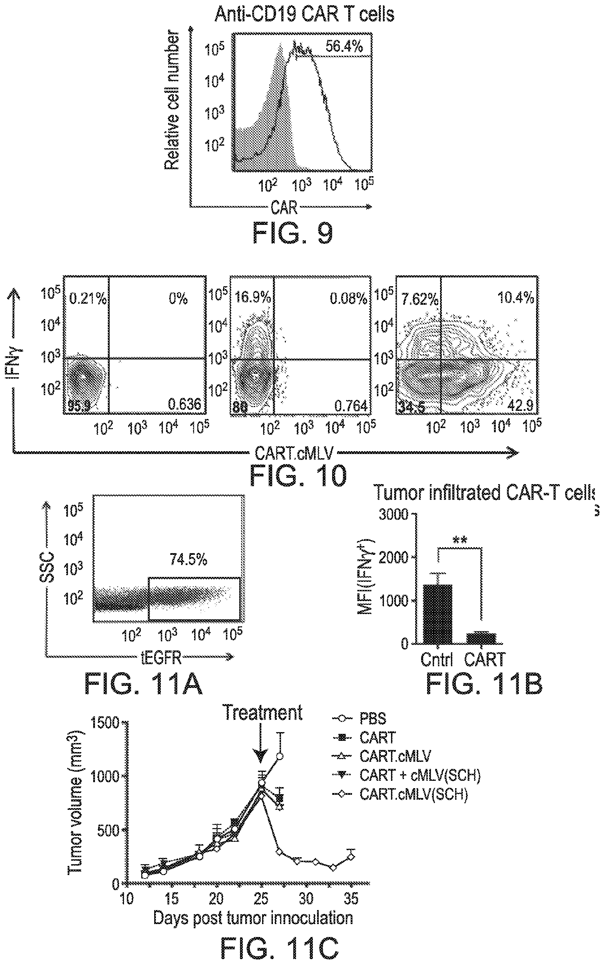

**Figure 9** depicts anti-CD19 CAR was stably expressed on the T cell surface. Flow cytometry analysis shows an average of 50% anti-CD19 CAR expression over total CD3⁺ T cells. The CAR-T cells were stained with rabbit anti-HA and followed by Alex647-anti-rabbit for CAR detection.
**Figure 10** depicts conjugation of cMLV did not alter CAR-T cell function. Representative flow cytometry analysis of IFNγ⁺CD3⁺ T cells, which were conjugated with DiD-labeled cMLV to distinguish the cMLV conjugated population. CART and CART.cMLV(DiD) were co-cultured with SKOV3.CD19 for 6 hours, and intracellular IFN-γ expression was quantified.

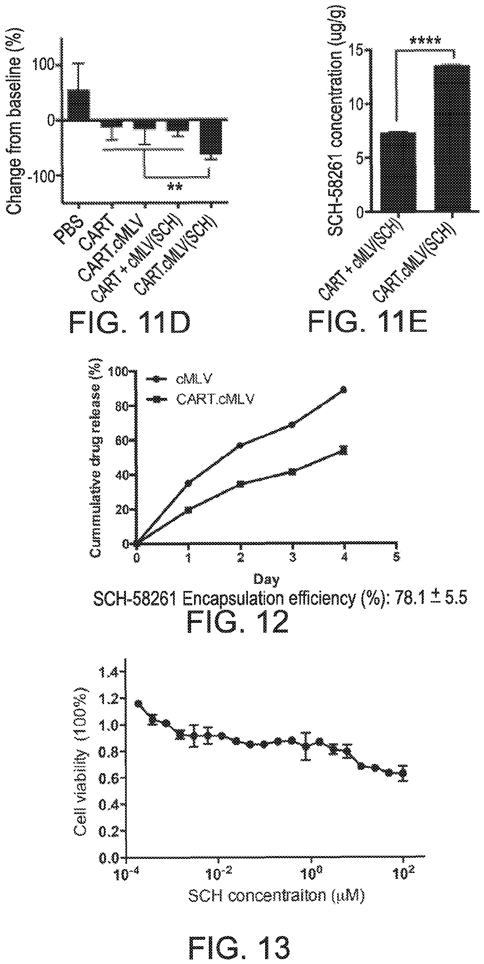

**Figure 11A** shows a representative flow cytometry analysis of C ART. tEGFR. cells stained with APC-anti-hEGFR shows tEGFR is stably expressed on *in vitro* culture of CAR.tEGFR cells 14 days post-transduction. **Figure 11B** depicts tumor infiltrated-T cells showed reduced IFN-γ secretion. Ten days post CAR-T cell therapy, tumor infiltrated T cells were stimulated *ex vivo* with anti-hCD3 and anti-hCD28. IFN-γ release was measured with intracellular staining. The positive control was spleenocytes of non-tumor bearing mice that received CAR-T cell infusion (n = 3, mean ± SD; ns, not significant; ^{∗}p < 0.05; ^{∗∗}p < 0.01; ^{∗∗∗}p < 0.001). **Figure 11C** depicts tumor progression was measured with a digital caliper. **Figure 11D** depicts CART.cMLV(SCH)showed significant reduction of tumor size 48 hours after treatment. The summarized statistics of each treatment group at 48 hours post-therapy is shown in bar graphs, (n = 10, mean ± SD; ns, not significant; ^{∗}p < 0.05; ^{∗∗}p < 0.01; ^{∗∗∗}p < 0.001). **Figure 11E** depicts HPLC analysis of intratumoral SCH-58261 concentrations (ug/g) in mice treated with SCH, including the groups CART + cMLV(SCH), and CART.cMLV(SCH).
**Figure 12** depicts *in vitro* release rates (%) of SCH58261 in cMLVs either unconjugated (cMLV) or conjugated to CAR-T cells (CART.cMLV). Error bars represent the standard deviation of the mean of triplicate experiments.
**Figure 13** depicts the cytotoxicity of cMLVs loaded with SCH58261 against SKOV-3 human ovarian cancer cells in vitro. Error bars represent the standard deviation of the mean of triplicate experiments.

### DETAILED DESCRIPTION OF THE INVENTION

Unless defined otherwise, technical and scientific terms used herein have the same meaning as commonly understood by one of ordinary skill in the art to which this invention belongs. Allen et al., Remington: The Science and Practice of Pharmacy 22nd ed., Pharmaceutical Press (September 15, 2012); Hornyak et al., Introduction to Nanoscience and Nanotechnology, CRC Press (2008); Singleton and Sainsbury, Dictionary of Microbiology and Molecular Biology 3rd ed., revised ed., J. Wiley & Sons (New York, NY 2006); Smith, March's Advanced Organic Chemistry Reactions, Mechanisms and Structure 7th ed., J. Wiley & Sons (New York, NY 2013); Singleton, Dictionary of DNA and Genome Technology 3rd ed., Wiley-Blackwell (November 28, 2012); and Green and Sambrook, Molecular Cloning: A LaboratoryManual 4th ed., Cold Spring Harbor Laboratory Press (Cold Spring Harbor, NY 2012), provide one skilled in the art with a general guide to many of the terms used in the present application. For references on how to prepare antibodies, see Greenfield, Antibodies A Laboratory Manual 2nd ed., Cold Spring Harbor Press (Cold Spring Harbor NY, 2013); Köhler and Milstein, Derivation of specific antibody-producing tissue culture arid tumor lines by cell fusion, Eur. J. Immunol. 1976 Jul, 6(7):511-9; Queen and Selick, Humanized immunoglobulins, U. S. Patent No. 5,585,089 (1996 Dec); and Riechmann et al., Reshaping human antibodies for therapy, Nature 1988 Mar 24, 332(6162):323-7.. convenience, certain terms employed herein, in the specification, examples and appended claims are collected here.

Unless stated otherwise, or implicit from context, the following terms and phrases include the meanings provided below. Unless explicitly stated otherwise, or apparent from context, the terms and phrases below do not exclude the meaning that the term or phrase has acquired in the art to which it pertains. The definitions are provided to aid in describing particular embodiments, and are not intended to limit the claimed invention, because the scope of the invention is limited only by the claims. Unless otherwise defined, all technical and scientific terms used herein have the same meaning as commonly understood by one of ordinary skill in the art to which this invention belongs.

As used herein the term "comprising" or "comprises" is used in reference to compositions, methods, and respective component(s) thereof, that are useful to an embodiment, yet open to the inclusion of unspecified elements, whether useful or not. It will be understood by those within the art that, in general, terms used herein are generally intended as "open" terms (e.g., the term "including" should be interpreted as "including but not limited to," the term "having" should be interpreted as "having at least," the term "includes" should be interpreted as "includes but is not limited to," etc.).

Unless stated otherwise, the terms "a" and "an" and "the" and similar references used in the context of describing a particular embodiment of the application (especially in the context of claims) can be construed to cover both the singular and the plural. The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (for example, "such as") provided with respect to certain embodiments herein is intended merely to better illuminate the application and does not pose a limitation on the scope of the application otherwise claimed. The abbreviation, "e.g." is derived from the Latin exempli gratia, and is used herein to indicate a non-limiting example. Thus, the abbreviation "e.g." is synonymous with the term "for example." No language in the specification should be construed as indicating any non-claimed element essential to the practice of the application.

As used herein, the term "about" refers to a measurable value such as an amount, a time duration, and the like, and encompasses variations of ±20%, ±10%, ±5%, ±1%, ±0.5% or ±0.1% from the specified value.

"Chimeric antigen receptor" or "CAR" or "CARs" as used herein refers to engineered receptors, which graft an antigen specificity onto cells (for example, T cells such as naïve T cells, central memory T cells, effector memory T cells or combination thereof). CARs are also known as artificial T-cell receptors, chimeric T-cell receptors or chimeric immunoreceptors. In various embodiments, CARs are recombinant polypeptides comprising an antigen-specific domain (ASD), a hinge region (HR), a transmembrane domain (TMD), co-stimulatory domain (CSD) and an intracellular signaling domain (ISD).

"Effector function" refers to the specialized function of a differentiated cell. Effector function of a T-cell, for example, may be cytolytic/cytotoxicity activity or helper activity including the secretion of cytokines.

"Genetically modified cells", "redirected cells", "genetically engineered cells" or "modified cells" as used herein refer to cells that express the CAR.

"Disease targeted by genetically modified cells" as used herein encompasses the targeting of any cell involved in any manner in any disease by the genetically modified cells of the invention, irrespective of whether the genetically modified cells target diseased cells or healthy cells to effectuate a therapeutically beneficial result. The genetically modified cells include but are not limited to genetically modified T-cells, NK cells, hematopoietic stem cells, pluripotent embryonic stem cells or embryonic stem cells. The genetically modified cells comprise crosslinked mulilayer liposome (CMLV), which encapsulates an A2A receptor inhibitor, and polynucleotides encoding one or more CARs. Examples of antigens which may be targeted include but are not limited to antigens expressed on B-cells; antigens expressed on carcinomas, sarcomas, lymphomas, leukemia, germ cell tumors, and blastomas; antigens expressed on various immune cells; and antigens expressed on cells associated with various hematologic diseases, autoimmune diseases, and/or inflammatory diseases. Other antigens that may be targeted will be apparent to those of skill in the art and may be targeted by the CARs of the invention in connection with alternate embodiments thereof.

"Immune cell" as used herein refers to the cells of the mammalian immune system including but not limited to antigen presenting cells, B-cells, basophils, cytotoxic T-cells, dendritic cells, eosinophils, granulocytes, helper T-cells, leukocytes, lymphocytes, macrophages, mast cells, memory cells, monocytes, natural killer cells, neutrophils, phagocytes, plasma cells and T-cells.

"Immune response" as used herein refers to immunities including but not limited to innate immunity, humoral immunity, cellular immunity, immunity, inflammatory response, acquired (adaptive) immunity, autoimmunity and/or overactive immunity.

As used herein, "CD4 lymphocytes" refer to lymphocytes that express CD4, i.e., lymphocytes that are CD4÷. CD4 lymphocytes may be T cells that express CD4.

The terms "T-cell" and "T-lymphocyte" are interchangeable and used synonymously herein. Examples include but are not limited to naïve T cells, central memory T cells, effector memory T cells or combinations thereof.

As used herein, the term "antibody" refers to an intact immunoglobulin or to a monoclonal or polyclonal antigen-binding fragment with the Fc (crystallizable fragment) region or FcRn binding fragment of the Fc region, referred to herein as the "Fc fragment" or "Fc domain". Antigen-binding fragments may be produced by recombinant DNA techniques or by enzymatic or chemical cleavage of intact antibodies. Antigen-binding fragments include, inter alia, Fab, Fab', F(ab')2, Fv, dAb, and complementarity determining region (CDR) fragments, single-chain antibodies (scFv), single domain antibodies, chimeric antibodies, diabodies and polypeptides that contain at least a portion of an immunoglobulin that is sufficient to confer specific antigen binding to the polypeptide. The Fc domain includes portions of two heavy chains contributing to two or three classes of the antibody. The Fc domain may be produced by recombinant DNA techniques or by enzymatic (e.g. papain cleavage) or via chemical cleavage of intact antibodies.

"Therapeutic agents" as used herein refers to agents that are used to, for example, treat, inhibit, prevent, mitigate the effects of, reduce the severity of, reduce the likelihood of developing, slow the progression of and/or cure, a disease. Diseases targeted by the therapeutic agents include but are not limited to infectious diseases, carcinomas, sarcomas, lymphomas, leukemia, germ cell tumors, blastoinas, antigens expressed on various immune cells, and antigens expressed on cells associated with various hematologic diseases, and/or inflammatory diseases.

"Cancer" and "cancerous" refer to or describe the physiological condition in mammals that is typically characterized by unregulated cell growth. The term "cancer" is meant to include all types of cancerous growths or oncogenic processes, metastatic tissues or malignantly transformed cells, tissues, or organs, irrespective of histopathologic type or stage of invasiveness. Examples of solid tumors include malignancies, e.g., sarcomas, adenocarcinomas, and carcinomas, of the various organ systems, such as those affecting liver, lung, breast, lymphoid, gastrointestinal (e.g., colon), genitourinary tract (e.g., renal, urothelial cells), prostate and pharynx. Adenocarcinomas include malignancies such as most colon cancers, rectal cancer, renal-cell carcinoma, liver cancer, non-small cell carcinoma of the lung, cancer of the small intestine and cancer of the esophagus. In one embodiment, the cancer is a melanoma, e.g., an advanced stage melanoma. Metastatic lesions of the aforementioned cancers can also be treated or prevented using the methods and compositions of the invention. Examples of other cancers that can be treated include bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin Disease, non-Hodgkin lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukemias including acute myeloid leukemia, chronic myeloid leukemia, acute lymphoblastic leukemia, chronic lymphocytic leukemia, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers including those induced by asbestos, and combinations of said cancers.

"Polynucleotide" as used herein includes but is not limited to DNA, RNA, cDNA (complementary DNA), mRNA (messenger RNA), rRNA (ribosomal RNA), shRNA (small hairpin RNA), snRNA (small nuclear RNA), snoRNA (short nucleolar RNA), miRNA (microRNA), genomic DNA, synthetic DNA, synthetic RNA, and/or tRNA.

The terms "T-cell" and "T-lymphocyte" are interchangeable and used synonymously herein. Examples include but are not limited to naïve T cells, central memory T cells, effector memory T cells or combinations thereof.

As used herein, the term "administering," refers to the placement an agent as disclosed herein into a subject by a method or route which results in at least partial localization of the agents at a desired site.

"Beneficial results" may include, but are in no way limited to, lessening or alleviating the severity of the disease condition, preventing the disease condition from worsening, curing the disease condition, preventing the disease condition from developing, lowering the chances of a patient developing the disease condition and prolonging a patient's life or life expectancy. As non-limiting examples, "beneficial results" or "desired results" may be alleviation of one or more symptom(s), diminishment of extent of the deficit, stabilized (i.e., not worsening) state of cancer progression, delay or slowing of metastasis or invasiveness, and amelioration or palliation of symptoms associated with the cancer.

As used herein, the terms "treat," "treatment," "treating," or "amelioration" refer to therapeutic treatments, wherein the object is to reverse, alleviate, ameliorate, inhibit, slow down or stop the progression or severity of a condition associated with, a disease or disorder. The term "treating" includes reducing or alleviating at least one adverse effect or symptom of a condition, disease or disorder, such as cancer. Treatment is generally "effective" if one or more symptoms or clinical markers are reduced. Alternatively, treatment is "effective" if the progression of a disease is reduced or halted. That is, "treatment" includes not just the improvement of symptoms or markers, but also a cessation of at least slowing of progress or worsening of symptoms that would be expected in absence of treatment. Beneficial or desired clinical results include, but are not limited to, alleviation of one or more symptom(s), diminishment of extent of disease, stabilized (i.e., not worsening) state of disease, delay or slowing of disease progression, amelioration or palliation of the disease state, and remission (whether partial or total), whether detectable or undetectable. The term "treatment" of a disease also includes providing relief from the symptoms or side-effects of the disease (including palliative treatment). In some embodiments, treatment of cancer includes decreasing tumor volume, decreasing the number of cancer cells, inhibiting cancer metastases, increasing life expectancy, decreasing cancer cell proliferation, decreasing cancer cell survival, or amelioration of various physiological symptoms associated with the cancerous condition.

"Conditions" and "disease conditions," as used herein may include, cancers, tumors or infectious diseases. In exemplary embodiments, the conditions include but are in no way limited to any form of malignant neoplastic cell proliferative disorders or diseases. In exemplary embodiments, conditions include any one or more of kidney cancer, melanoma, prostate cancer, breast cancer, glioblastoma, lung cancer, colon cancer, or bladder cancer.

The term "effective amount" or "therapeutically effective amount" as used herein refers to the amount of a pharmaceutical composition comprising one or more peptides as disclosed herein or a mutant, variant, analog or derivative thereof, to decrease at least one or more symptom of the disease or disorder, and relates to a sufficient amount of pharmacological composition to provide the desired effect. The phrase "therapeutically effective amount" as used herein means a sufficient amount of the composition to treat a disorder, at a reasonable benefit/risk ratio applicable to any medical treatment.

A therapeutically or prophylactically significant reduction in a symptom is, e.g. at least about 10%, at least about 20%, at least about 30%, at least about 40%, at least about 50%, at least about 60%, at least about 70%, at least about 80%, at least about 90%, at least about 100%, at least about 125%, at least about 150% or more in a measured parameter as compared to a control or non-treated subject or the state of the subject prior to administering the oligopeptides described herein. Measured or measurable parameters include clinically detectable markers of disease, for example, elevated or depressed levels of a biological marker, as well as parameters related to a clinically accepted scale of symptoms or markers for diabetes. It will be understood, however, that the total daily usage of the compositions and formulations as disclosed herein will be decided by the attending physician within the scope of sound medical judgment. The exact amount required will vary depending on factors such as the type of disease being treated, gender, age, and weight of the subject.

"Mammal" as used herein refers to any member of the class *Mammalia*, including, without limitation, humans and nonhuman primates such as chimpanzees and other apes and monkey species; farm animals such as cattle, sheep, pigs, goats and horses; domestic mammals such as dogs and cats; laboratory animals including rodents such as mice, rats and guinea pigs, and the like. The term does not denote a particular age or sex. Thus, adult and newborn subjects, as well as fetuses, whether male or female, are intended to be included within the scope of this term.

"Particle" as used herein refers to particulate matters of various sizes and any shape. The appropriate particle size can vary based on the materials used to make the particle, the active agent or therapeutic agent earned therein, and the functional groups and chemistry involved for conjugation with an immune effector cell, as will be appreciated by a person of skill in the art in light of the teachings disclosed herein. For example, the particles can be nanoparticles having an averaged diameter between 1 nm and 1,000 nm, or microparticles having an averaged diameter greater than 1µm but about at least an order of magnitude smaller than the immune effector cell to which the particles conjugated. For example, in some embodiments the particle has a diameter of from about 1 nm to about 1000 nm; or from about 25 nm to about 750 nm; or from about 50 nm to about 500 nm; or from about 100 nm to about 300 nm. In some embodiments, the average particle size can be about 1 nm, about 10 nm, about 50 nm, about 100 nm, about 150 nm, about 200 nm, about 250 nm, about 300 nm, about 350 nm, about 400 nm, about 450 nm, about 500 nm, about 550 nm, about 600 nm, about 650 nm, about 700 nm, about 750 nm, about 800 nm, about 850 nm, about 900 nm, about 950 nm, or about 1000 nm, or about 2,000 nm, or about 5,000 nm, or about 6,000 nm, or about 10,000 nm. In some embodiments, the particle can be a nanoparticle or a microparticle, as these terms are defined herein. The particles can be all nanoparticles, all microparticles, or a combination of nanoparticles and microparticles. In some embodiments, the particles are liposomes. In other embodiments, the particles are polymeric particles formed from biocompatible and/or biodegradable polymers. In some embodiments, the particles contain a core. In some embodiments, the particles contain a coating.

"Biodegradable polymer" as used herein can contain a synthetic polymer, although natural polymers also can be used. The polymer can be, for example, poly(lactic-co-glycolic acid) (PLGA), polystyrene or combinations thereof. The polystyrene can, for example, be modified with carboxy groups. Other examples of biodegradable polymers include poly(hydroxy acid); poly(lactic acid); poly(glycolic acid); poly(lactic acid-co-glycolic acid); poly(lactide); poly(glycolide); poly(1actide-co-glycolide); polyanhydrides; polyorthoesters; polyamides; polycarbonates; polyalkylenes; polyethylene; polypropylene; polyalkylene glycols; poly(ethylene glycol); polyalkylene oxides; poly(ethylene oxides); polyalkylene terephthalates; poly(ethylene terephthalate); polyvinyl alcohols; polyvinyl ethers; polyvinyl esters; polyvinyl halides; poly(vinyl chloride); polyvinylpyrrolidone; polysiloxanes; poly(vinyl alcohols); poly(vinyl acetate); polyurethanes; co-polymers of polyurethanes; derivativized celluloses; alkyl cellulose; hydroxyalkyl celluloses; cellulose ethers; cellulose esters; nitro celluloses; methyl cellulose; ethyl cellulose; hydroxypropyl cellulose; hydroxy-propyl methyl cellulose; hydroxybutyl methyl cellulose; cellulose acetate; cellulose propionate; cellulose acetate butyrate; cellulose acetate phthalate; carboxylethyl cellulose; cellulose triacetate; cellulose sulfate sodium salt; polymers of acrylic acid; methacrylic acid; copolymers of methacrylic acid; derivatives of methacrylic acid; poly(methyl methacrylate); poly(ethyl methacrylate); poly(butylmethacrylate); poly(isobutyl methacrylate); poly(hexylmethacrylate); poly(isodecyl methacrylate); polyflauryl methacrylate); poly(phenyl methacrylate); poly(methyl acrylate); poly(isopropyl acrylate); poly(isobutyl acrylate); poly(octadecyl acrylate); poly(butyric acid); poly(valeric acid); poly(lactide-co-caprolactone); copolymers of poly(lactide-co-caprolactone); blends of poly(lactide-co-caprolactone); poly-(isobutyl cyanoacrylate); poly(2-hydroxyethyl-L-glutam-nine); and combinations, copolymers and/or mixtures of one or more of any of the foregoing. Furthermore, as a person of ordinary skill in the art would appreciate, some of the polymers listed above as "biocompatible" can also be considered biodegradable, whether or not they are included in the above listing of representative biodegradable polymers. As used herein, "derivatives" include polymers having substitutions, additions of chemical groups and other modifications routinely made by those skilled in the art.

Adoptive T cell transfers of genetically modified cytotoxic T cells to express chimeric antigen receptors (CAR) have become a promising immunotherapy method. Multiple researcher studies have shown that adoptive transfer of CAR T cells is successful in patients with B cell hematological malignancies, but is still in the earlier stages of development for treatment of solid tumors. One limiting factor to adoptive T cell therapy is the suppressive tumor microenvironment that inactivates tumor infiltrated T cell (TIL) function. The tumor microenvironment contains high concentration of TIL suppressor molecules such as adenosine that is up taken by the A2A receptor expressed on the cell surface of CD4 and CD8 T cells. Adenosine is generated from extracellular ATP through CD39 and CD73 expressed on the surface of tumor cells and regulatory T cells. The inventors have demonstrated that co-delivery of CAR T cells conjugated with crosslinked multilayer liposome vesicles (cMLV) encapsulating the A2A receptor inhibitor prevented reduction of CART cell effector function in the tumor microenvironment and effectively restricted tumor growth. The inventors further investigated the application of CAR T cells as chaperones for drug encapsulating cMLVs to rescue hypofunctional T cells residing in the tumor. In this in vivo study, the inventors showed that the "rescue" system was able to recover TIL function, increase tumor infiltrating CD3+ T cells and decrease the tumor size within 48-h post treatment.

An engineered cell is provided, where active agent-loaded particles are chemically conjugated to the surface of the cell. In various embodiments, the engineered cell is an engineered immune effector cell (e.g., CAR T cell, B cell, natural killer cell, hematopoietic stem cell or tumor-specific T lymphocyte). In various embodiments, nanoparticles or microparticles, encapsulating an active agent, are chemically bonded to the surface of the cell. Particles can be crosslinked multilayer liposomes (cMLVs) or polymeric particles.

In some embodiments, an engineered immune effector cell is a CAR-expressing T cell (CAR T cell), with cMLVs conjugated at the surface, where the cMLVs encapsulate an inhibitor of A2aR. In other embodiments, an engineered immune effector cell is a T cell containing polynucleotides which encode one or more CARs, and the T cell is conjugated at the surface with cMLVs, where the cMLV encapsulates an A2a receptor inhibitor. In some embodiments, the A2A receptor inhibitor is SCH58261.

Generally, nanoparticles are conjugated to each cell at a ratio that does not alter the function of the cell, yet high enough to deliver a high load of active agent per cell. For example, the number of conjugated nanoparticles per cell is between 400 and 350, between 350 and 300, between 300 and 250, between 250 and 200, between 200 and 150, or between 150 and 100.

Also provided herein are methods for treating, inhibiting and/or reducing severity or likelihood of a disease in a subject in need thereof. The methods comprise administering to the subject a therapeutically effective amount of a composition comprising immune effector cells comprising crosslinked mulilayer liposome (cMLV) encapsulating an A2A receptor inhibitor so as to treat, inhibit and/or reduce the severity or likelihood of the disease in the subject. In some embodiments, the immune effector cells include NK cells, T cells (including CAR-expressing T cells), tumor-specific T lymphocytes and/or hematopoietic stem cells. In various embodiments, the immune effector cells contain polynucleotides that encode CARs. In some embodiments, the A2A receptor inhibitor is SCH58261, caffeine or ZM241385. In some embodiments, the disease is associated with the antigen targeted by the CAR in the composition.

Provided herein are methods for treating, inhibiting, reducing the severity and/or likelihood of metastasis of cancer in a subject in need thereof. The methods comprise administering to the subject a therapeutically effective amount of a composition comprising immune effector cells comprising crosslinked mulilayer liposome (cMLV) encapsulating an A2a receptor inhibitor, so as to treat, inhibit, reduce the severity and/or likelihood of metastasis of cancer in the subject. In some embodiments, the A2A receptor inhibitor is SCH58261, caffeine, SYN115, FSPTP, ZM241385, PBS-509, ST1535, ST4206, Tozadenant, V81444, or Istradefylline.

In various embodiments, the subject in need of any of the treatment methods above is one having pre-existing TILs or having previously received CAR-T cell therapy.

In various embodiments, any of the methods above, following administration of the composition to a subject, further features one or more of the following: an average density of infiltrated T cells in the tumor between 0.20 and 0.25 cells/mm², between 0.25 and 0.30 cells/mm², between 0.30 and 0.35 cells/mm², between 0.35 and 0.40 cells/mm² or greater; a reduction in tumor size by about 10%, 20%, 30%, 40%, 50%, 60% or greater; a total T cell population in the tumor of at least 5%, 6%, 7%, 8%, 9%, 10%, 11%, 12%, 13%, 14%, 15% or greater; as compared to control groups such as a subject not administered, a subject administered with immune effector cells that lack the surface-conjugated nanoparticles or active agents, a subject administered with immune effector cells that are surface-conjugated with nanoparticles but no A2aR inhibitors, a subject administered with both immune effector cells and nanoparticles even encapsulating an A2aR inhibitor, or a subject prior to the administration of immune effector cells that are surface conjugated with nanoparticles which incorporate an A2aR inhibitor.

In various embodiments, the disease to be treated, inhibited or reduced severity or likelihood of in any of the methods above includes cancers, and in some embodiments, CD73-expressing tumors.

In various embodiments, the antigens targeted by the CARs include but are not limited to any one or more of 4-1BB, 5T4, adenocarcinoma antigen, alpha-fetoprotein, BAFF, β-lymphoma cell, C242 antigen, CA-125, carbonic anhydrase 9 (CA-IX), C-MET, CCR4, CD152, CD19, CD20, CD200, CD22, CD221, CD23 (IgE receptor), CD28, CD30 (TNFRSF8), CD33, CD4, CD40, CD44 v6, CD51, CD52, CD56, CD74, CD80, CEA, CNT0888, CTLA-4, DR5, EGFR, EpCAM, CD3, FAP, fibronectin extra domain-B, folate receptor 1, GD2, GD3 ganglioside, glycoprotein 75, GPNMB, HER2/neu, HGF, human scatter factor receptor kinase, IGF-1 receptor, IGF-I, IgGl, L1-CAM, IL-13, IL-6, insulin-like growth factor I receptor, integrin α5β1, integrin αvβ3, MORAb-009, MS4A1, MUC1, mucin CanAg, N-glycolylneuraminic acid, NPC-1C, PDGF-R α, PDL192, phosphatidylserine, prostatic carcinoma cells, RANKL, RON, ROR1, SCH 900105, SDC1, SLAMF7, TAG-72, tenascin C, TGF beta 2, TGF-β, TRAIL-R1, TRAIL-R2, tumor antigen CTAA16.88, VEGF-A, VEGFR-1, VEGFR2 or vimentin. Other antigens specific for cancer will be apparent to those of skill in the art and may be used in connection with alternate embodiments of the invention.

In some embodiments, the therapeutic methods described herein further comprise administering to the subject, sequentially or simultaneously, existing therapies. Examples of existing cancer treatment include, but are not limited to, active surveillance, observation, surgical intervention, chemotherapy, immunotherapy, radiation therapy (such as external beam radiation, stereotactic radiosurgery (gamma knife), and fractionated stereotactic radiotherapy (FSR)), focal therapy, systemic therapy, vaccine therapies, viral therapies, molecular targeted therapies, or combinations thereof.

Examples of chemotherapeutic agents include but are not limited to Albumin-bound paclitaxel (nab-paclitaxel), Actinomycin, Alitretinoin, All-trans retinoic acid, Azacitidine, Azathioprine, Bevacizumab, Bexatotene, Bleomycin, Bortezomib, Carboplatin, Capecitabine, Cetuximab, Cisplatin, Chlorambucil, Cyclophosphamide, Cytarabine, Daunorubicin, Docetaxel, Doxifluridine, Doxorubicin, Epirubicin, Epothilone, Erlotinib, Etoposide, Fluorouracil, Gefitinib, Gemcitabine, Hydroxyurea, Idarubicin, Imatinib, Ipilimumab, Irinotecan, Mechlorethamine, Melphalan, Mercaptopurine, Methotrexate, Mitoxantrone, Ocrelizumab, Ofatumumab, Oxaliplatin, Paclitaxel, Panitumab, Pemetrexed, Rituximab, Tafluposide, Teniposide, Tioguanine, Topotecan, Tretinoin, Valrubicin, Vemurafenib, Vinblastine, Vincristine, Vindesine, Vinorelbine, Vorinostat, Romidepsin, 5-fluorouracil (5-FU), 6-mercaptopurine (6-MP), Cladribine, Clofarabine, Floxuridine, Fludarabine, Pentostatin, Mitomycin, ixabepilone, Estramustine, or a combination thereof.

In various embodiments, the therapeutically effective amount of the A2a receptor inhibitor is any one or more of about 0.01 to 0.05.µg/kg/day, 0.05-0.1µg/kg/day, 0.1 to 0.5.µg/kg/day, 0.5 to 5 µg/kg/day, 5 to 10 µg/kg/day, 10 to 20 µg/kg/day, 20 to 50 µg/kg/day, 50 to 100 µg/kg/day, 100 to 150 µg/kg/day, 150 to 200 µg/kg/day, 200 to 250 µg/kg/day, 250 to 300 µg/kg/day, 300 to 350 µg/kg/day, 350 to 400 µg/kg/day, 400 to 500 µg/kg/day, 500 to 600 µg/kg/day, 600 to 700µg/kg/day, 700 to 800µg/kg/day, 800 to 900µg/kg/day, 900 to 1000 µg/kg/day, 0.01 to 0.05mg/kg/day, 0.05-0.1mg/kg/day, 0.1 to 0.5mg/kg/day, 0.5 to 1 mg/kg/day, 1 to 5 mg/kg/day, 5 to 10 mg/kg/day, 10 to 15 mg/kg/day, 15 to 20 mg/kg/day, 20 to 50 mg/kg/day, 50 to 100 mg/kg/day, 100 to 200 mg/kg/day, 200 to 300 mg/kg/day, 300 to 400 mg/kg/day, 400 to 500 mg/kg/day, 500 to 600 mg/kg/day, 600 to 700mg/kg/day, 700 to 800mg/kg/day, 800 to 900mg/kg/day, 900 to 1000 mg/kg/day or a combination thereof. Typical dosages of an effective amount of the A2A receptor described herein can be in the ranges recommended by the manufacturer where known therapeutic compounds are used, and also as indicated to the skilled artisan by the *in vitro* responses or responses in animal models. Such dosages typically can be reduced by up to about an order of magnitude in concentration or amount without losing relevant biological activity. The actual dosage can depend upon the judgment of the physician, the condition of the patient, and the effectiveness of the therapeutic method based, for example, on the *in vitro* responsiveness of relevant cultured cells or histocultured tissue sample, such as biopsied malignant tumors, or the responses observed in the appropriate animal models.

When "an immunologically effective amount," "an anti-tumor effective amount," "a tumor-inhibiting effective amount," or "therapeutically effective amount" is indicated, the precise amount of the compositions of the present invention to be administered can be determined by a physician with consideration of individual differences in age, weight, tumor size, extent of infection or metastasis, and condition of the patient (subject). It can generally be stated that a pharmaceutical composition comprising the immune effector cells (e.g., T cells, NK cells) described herein may be administered at a dosage of 10⁴ to 10⁹ cells/kg body weight, in some instances 10⁵ to 10⁶ cells/kg body weight, including all integer values within those ranges. T cell compositions may also be administered multiple times at these dosages. The cells can be administered by using infusion techniques.

In certain aspects, it may be desired to administer composition comprising immune effector cells (e.g., T cells, NK cells) described herein to a subject and then subsequently redraw blood (or have an apheresis performed), activate immune effector cells (e.g., T cells, NK cells) therefrom according to the present invention, and reinfuse the patient with these activated and expanded immune effector cells (e.g., T cells, NK cells). This process can be carried out multiple times every few weeks. In certain aspects, immune effector cells (e.g., T cells, NK cells) can be activated from blood draws of from ICcc to 400cc. In certain aspects, immune effector cells (e.g., T cells, NK cells) are activated from blood draws of 20cc, 30cc, 40cc, 50cc, 60cc, 70cc, 80cc, 90cc, or 100cc.

In various embodiments, the compositions of the invention comprising crosslinked multilayer liposome (CMLV), which encapsulates an A2A receptor inhibitor, and polynucleotides encoding one or more CARs described herein may be administered once a day (SID/QD), twice a day (BID), three times a day (TID), four times a day (QID), or more, so as to administer an effective amount of the composition to the subject, where the effective amount is any one or more of the doses described herein.

### Pharmaceutical Composition

In various embodiments, the present invention provides a pharmaceutical composition. The pharmaceutical composition includes immune effector cells comprising crosslinked multilayer liposome (CMLV) encapsulating an A2A receptor inhibitor. In some embodiments, the immune effector cells include NK cells, T cells (including CAR-expressing T cells), tumor-specific T lymphocytes and/or hematopoietic stem cells. In some embodiments, the A2A receptor inhibitor is SCH58261, caffeine or ZM241385.

The pharmaceutical compositions according to the invention can contain any pharmaceutically acceptable excipient. "Pharmaceutically acceptable excipient" means an excipient that is useful in preparing a pharmaceutical composition that is generally safe, nontoxic, and desirable, and includes excipients that are acceptable for veterinary use as well as for human pharmaceutical use. Such excipients may be solid, liquid, semisolid, or, in the case of an aerosol composition, gaseous. Examples of excipients include but are not limited to starches, sugars, microcrystalline cellulose, diluents, granulating agents, lubricants, binders, disintegrating agents, wetting agents, emulsifiers, coloring agents, release agents, coating agents, sweetening agents, flavoring agents, perfuming agents, preservatives, antioxidants, plasticizers, gelling agents, thickeners, hardeners, setting agents, suspending agents, surfactants, humectants, carriers, stabilizers, and combinations thereof.

In various embodiments, the pharmaceutical compositions according to the invention may be formulated for delivery via any route of administration. "Route of administration" may refer to any administration pathway known in the art, including but not limited to aerosol, nasal, oral, transmucosal, transdermal, parenteral or enteral. "Parenteral" refers to a route of administration that is generally associated with injection, including intraorbital, infusion, intraarterial, intracapsular, intracardiac, intradermal, intramuscular, intraperitoneal, intrapulmonary, intraspinal, intrasternal, intrathecal, intrauterine, intravenous, subarachnoid, subcapsular, subcutaneous, transmucosal, or transtracheal. Via the parenteral route, the compositions may be in the form of solutions or suspensions for infusion or for injection, or as lyophilized powders. Via the parenteral route, the compositions may be in the form of solutions or suspensions for infusion or for injection. Via the enteral route, the pharmaceutical compositions can be in the form of tablets, gel capsules, sugar-coated tablets, syrups, suspensions, solutions, powders, granules, emulsions, microspheres or nanospheres or lipid vesicles or polymer vesicles allowing controlled release. Typically, the compositions are administered by injection. Methods for these administrations are known to one skilled in the art.

The pharmaceutical compositions according to the invention can contain any pharmaceutically acceptable carrier. "Pharmaceutically acceptable carrier" as used herein refers to a pharmaceutically acceptable material, composition, or vehicle that is involved in carrying or transporting a compound of interest from one tissue, organ, or portion of the body to another tissue, organ, or portion of the body. For example, the carrier may be a liquid or solid filler, diluent, excipient, solvent, or encapsulating material, or a combination thereof. Each component of the carrier must be "pharmaceutically acceptable" in that it must be compatible with the other ingredients of the formulation. It must also be suitable for use in contact with any tissues or organs with which it may come in contact, meaning that it must not carry a risk of toxicity, irritation, allergic response, immunogenicity, or any other complication that excessively outweighs its therapeutic benefits.

The pharmaceutical compositions according to the invention can also be encapsulated, tableted or prepared in an emulsion or syrup for oral administration. Pharmaceutically acceptable solid or liquid carriers may be added to enhance or stabilize the composition, or to facilitate preparation of the composition. Liquid carriers include syrup, peanut oil, olive oil, glycerin, saline, alcohols and water. Solid carriers include starch, lactose, calcium sulfate, dihydrate, terra alba, magnesium stearate or stearic acid, talc, pectin, acacia, agar or gelatin. The carrier may also include a sustained release material such as glyceryl monostearate or glyceryl di stearate, alone or with a wax.

The pharmaceutical preparations are made following the conventional techniques of pharmacy involving milling, mixing, granulation, and compressing, when necessary, for tablet forms; or milling, mixing and filling for hard gelatin capsule forms. When a liquid carrier is used, the preparation will be in the form of a syrup, elixir, emulsion or an aqueous or non-aqueous suspension. Such a liquid formulation may be administered directly p.o. or filled into a soft gelatin capsule.

The pharmaceutical compositions according to the invention may be delivered in a therapeutically effective amount. The precise therapeutically effective amount is that amount of the composition that will yield the most effective results in terms of efficacy of treatment in a given subject. This amount will vary depending upon a variety of factors, including but not limited to the characteristics of the therapeutic compound (including activity, pharmacokinetics, pharmacodynamics, and bioavailability), the physiological condition of the subject (including age, sex, disease type and stage, general physical condition, responsiveness to a given dosage, and type of medication), the nature of the pharmaceutically acceptable carrier or carriers in the formulation, and the route of administration. One skilled in the clinical and pharmacological arts will be able to determine a therapeutically effective amount through routine experimentation, for instance, by monitoring a subject's response to administration of a compound and adjusting the dosage accordingly. For additional guidance, see Remington: The Science and Practice of Pharmacy (Gennaro ed. 20th edition, Williams & Wilkins PA, USA) (2000).

Before administration to patients, formulants may be added to the rAAV vector, the cell transfected with the rAAV vector, or the supernatant conditioned with the transfected cell. A liquid formulation may be preferred. For example, these formulants may include oils, polymers, vitamins, carbohydrates, amino acids, salts, buffers, albumin, surfactants, bulking agents or combinations thereof.

Carbohydrate formulants include sugar or sugar alcohols such as monosaccharides, disaccharides, or polysaccharides, or water soluble glucans. The saccharides or glucans can include fructose, dextrose, lactose, glucose, mannose, sorbose, xylose, maltose, sucrose, dextran, pullulan, dextrin, alpha and beta cyclodextrin, soluble starch, hydroxethyl starch and carboxymethylcellulose, or mixtures thereof. "Sugar alcohol" is defined as a C4 to C8 hydrocarbon having an -OH group and includes galactitol, inositol, mannitol, xylitol, sorbitol, glycerol, and arabitol. These sugars or sugar alcohols mentioned above may be used individually or in combination. There is no fixed limit to amount used as long as the sugar or sugar alcohol is soluble in the aqueous preparation. In one embodiment, the sugar or sugar alcohol concentration is between 1.0 w/v % and 7.0 w/v %, more preferable between 2.0 and 6.0 w/v %.

Amino acids formulants include levorotary (L) forms of carnitine, arginine, and betaine; however, other amino acids may be added.

In some embodiments, polymers as formulants include polyvinylpyrrolidone (PVP) with an average molecular weight between 2,000 and 3,000, or polyethylene glycol (PEG) with an average molecular weight between 3,000 and 5,000.

It is also preferred to use a buffer in the composition to minimize pH changes in the solution before lyophilization or after reconstitution. Most any physiological buffer may be used including but not limited to citrate, phosphate, succinate, and glutamate buffers or mixtures thereof. In some embodiments, the concentration is from 0.01 to 0.3 molar. Surfactants that can be added to the formulation are shown in EP Nos. 270,799 and 268,110.

Another drug delivery system for increasing circulatory half-life is the liposome. Methods of preparing liposome delivery systems are discussed in Gabizon et al., Cancer Research (1982) 42:4734; Cafiso, Biochem Biophys Acta (1981) 649:129; and Szoka, Ann Rev Biophys Eng (1980) 9:467. Other drug delivery systems are known in the art and are described in, e.g., Poznansky et al., DRUG DELIVERY SYSTEMS (R. L. Juliano, ed., Oxford, N.Y. 1980), pp. 253-315; M. L. Poznansky, Pharm Revs (1984) 36:277.

After the liquid pharmaceutical composition is prepared, it may be lyophilized to prevent degradation and to preserve sterility. Methods for lyophilizing liquid compositions are known to those of ordinary skill in the art. Just prior to use, the composition may be reconstituted with a sterile diluent (Ringer's solution, distilled water, or sterile saline, for example) which may include additional ingredients. Upon reconstitution, the composition is administered to subjects using those methods that are known to those skilled in the art.

### EXAMPLES

### Example 1

### Experimental Methods

### Materials

Human ovarian cancer cell line SKOV3 and human chronic myelogenous leukemia cell line K562 cell line were obtained from ATCC and maintained in RPMI-1640 with 10 % heat-inactivated FBS. CD19⁺ K562 and CD19⁺SKOV3 cells were generated by transducing parental K562 and SKOV3 cells with FUW-CD19 lentivirus and sorting for CD19⁺ cells by fluorescence-activated cell sorting (FACS). SCH58261 was purchased from Sigma-Aldrich (St. Louis, MO). All lipids were purchased from NOF Corporation (Japan): 1,2-dioleoyl-sn-glycero-3- phosphocholine (DOPC), 1,2-dioleoyl-sn-glycero-3-phospho-(10-rac-glycerol) (DOPG), and 1,2-dioleoyl-sn-glyeero-3-phosphoethanolamine-N-[4-(p-maleimidophenyl)but-yramide (maleimide-headgroup lipid, MPB-PE).

Female 6-10 weeks-old NOD/scid/IL2rγ-/- (NSG) mice were purchased from The Jackson laboratory (Bar Harbor, ME). All mice were held under specific pathogen-reduced conditions in the animal facility of the University of Southern California (Los Angeles, CA, USA). All experiments were performed in accordance with the guidelines set by the National Institute of Health and the University of Southern California on the Care and Use of Animals.

### Generation of anti-hCD19 plasmid construct

An FUW lentiviral vector was constructed to express an HA-tagged anti-CD19 second generation CAR protein. The anti-CD19 single-chain variable fragment (scFv) sequence was developed by Carl June (Patent Number US 2013/0287748 A1, 2013). This sequence, followed by the human CD8 hinge region (aa 138-184), was codon optimized and constructed by IDT DNA. The anti-CD19/CD8 hinge gene block was combined with the transmembrane and intracellular domains of human CD28 (aa 153-220) and the intracellular domain of human CD3ξ (aa 52-164) using PCR. An HA tag was inserted upstream of the anti-CD19 scFv (sequence: tacccatacgatgttccagattacgct) to allow for labeling of CAR expressing cells. A Kozac sequence and the human CD8 leader sequence were also inserted upstream of the CAR construct. To make the lentiviral vector, this sequence was inserted downstream of the human ubiquitin-C promoter in the lentiviral plasmid FUW using Gibson assembly.

### Viral Production

Lentiviral vectors were made by transiently transfecting 293T cells using a standard calcium phosphate precipitation method. 293T cells were seeded in 15 cm plates and transfected 14-18 hours later, once cells had reached a confluency of 80-90%. For transfection, 40 µg of the FUW-CAR plasmid was combined with 20 µg each of the packaging plasmids pMDLg/pRRE and pRSV-Rev and the pVSVg envelope plasmid. 4 hours after transfection cells, the media was removed, cells were washed with PBS, and new media was added. Viral supernatant was harvested 48 hours after transfection.

### Preparation of T cells for adoptive transfer and lentiviral transduction

Thawed peripheral blood mononuclear cells (PBMCs) from healthy donors were cultured in T cell medium (TCM) containing X-vivo15 serum free medium (Lonza), 5% (vol/vol) GemCell human serum antibody AB (Gemini bio-products, West Sacramento CA), 1% (vol/vol) Glutamax-100x (Gibco Life Technologies), 10nM HEPES buffer (Corning), 1% (vol/vol) penicillin/streptomycin (Corning) and 12.25 mM N-Acetyl-L-cysteine (Sigma). The culture is supplemented with 10ng/mL human IL-2. The PBMCs were activated using Dynabeads CD3/CD28 beads T cell expander (three beads per cell; Invitrogen) at the density of 10e+6 cells/mL and transduced with lentivirus two days post activation. During ex vivo expansion, culture medium was replenished, and the T cell density was maintained between 0.5-1 × 10⁶ cells/mL.The transduced cells were expanded and harvested on day 13-post activation.

### Detection of receptor expression on T cell surface

HA-tagged CD19scFv-28-ζ CAR-T cells were washed with PBS and stained with rabbit anti-HA followed by Alex647-conjugated anti-rabbit antibodies for CAR detection. Retrovirus-transduced cells were stained with APC-conjugated anti-human EGFR for tEGFR detection. Receptor expression was analyzed using the MACS Quant flow cytometry analyzer (Miltenyi Biotec, Inc., San Diego, CA).

### Synthesis of nanoparticles

Liposomes were prepared based on the conventional dehydration-rehydration method reported in Joo et a! (2013). To encapsulate SCH-58261 into cMLVs, 1 mg of SCH in organic solvent was mixed with the lipid mixture to form dried thin lipid films. To label liposome particles with DiD lipophilic dyes, DiD dyes were added to the lipid mixture in chloroform at a ratio of 0.01-.1 (DiD:lipids). Crosslinked multi-lamelar liposomes were prepared from 1.5 µmol of lipids DOPC: DOPG: MPE-PE = 40:10:50 mixed in chloroform and evaporated under argon gas before drying under vacuum overnight to form dried lipid films. The lipid film was rehydrated in 10mM Bis-Tris propane at pH 7.0. After the lipid was mixed, either with or without SCH58261, through vigorous vortexing every 10 minutes for 1h, they undergo 4 cycles of 15-second sonication (Misonix Microson XL2000, Farmingdale, NY) and rested on ice at 1-minute intervals after each cycle. A final concentration of 10mM MgCl₂ was added to induce divalent-triggered vesicle fusion. The crosslinking of multilamellar vesicles (cMLVs) were performed by addition of Dithiothreitol (DTT, Sigma-Aldrich) at a final concentration of 1.5 mM for 1h at 37°C. The crosslinked multilamellar vesicles were collected by centrifugation at 14,000 g for 4 minutes and washed twice with PBS. Morphological analysis of multilamellar structure of vesicles were performed and confirmed by cryo-electron microscopy in previous studies. The hydrodynamic size of cMLVs was measured by dynamic light scattering (Wyatt Technology, Santa Barbara, CA). The particles were suspended in filtered water, vortexed and sonicated prior to analysis. Detailed information on small molecule loading efficiency as well as release kinetics is included in the Supplementary Methods.

### Nanoparticle conjugation with cells and in situ PEGylation

Chemical conjugation of nanoparticles to the cells was performed based on a method provided in Stephan et al (2010). We resuspended 10 × 10⁶ cells/mL in serum free X-vivo 15 medium (Lonza). Then, equal volumes of nanoparticles were resuspended in nuclease free water at different nanoparticles to T cell conjugation ratios and incubated at 37°C. The cells and nanoparticles were mixed every 10 minutes for a total duration of 30 minutes. After a PBS wash to segregate unbound nanoparticles from cells, we incubated 10 × 10⁶ per mL with 1 mg ml⁻¹ thiol-terminated 2-kDa PEG at 37°C for 30 minutes in TCM to quench residual maleimide groups on cell-bound particles. We performed two PBS washes to remove unbound PEG.

For quantification of cell bound particles, particles were fluorescently labeled with the lipid-like fluorescent dye 1,1'-dioctadecyl-3,3,3',3'-tetramethylindodicarbocyanine (DiD) before conjugation, and fluorescence was detected the particle fluorescence with flow cytometry and fluorescent microplate reader. The surface conjugation of DiD-labeled cMLVs and CFSE-labeled CAR-T cells was further visualized using confocal microscopy.

### Cytotoxicity assay

The modified version of a cytotoxicity assay was performed as previously described Kochenderfer et al (2009). SKOV3 cells and K562 cells (non-target) were suspended in TCM at the concentration of 1.5 × 10⁶ cells/mL and stained with the fluorescent dye 5-(and-6)-(((4-chloromethyl)benzoyl)amino) tetramethylrhodamine (CMTMR) (Invitrogen) at the concentration of 5µM. The cells were mixed and incubated at 37°C for 30 minutes. The cells were washed, resuspended in TCM and incubated at 37°C for 60 minutes. Then, the cells were washed twice and resuspended in TCM. Target cells (SKOV3-CD19⁺ cells and K562-CD19⁺) were suspended in PBS+0.1% BSA at 1 × 10⁶ cells/mL and stained with carboxyfluorescein diacetate succinimide ester (CFSE) (Invitrogen) at the concentration 1 µM. The cells were incubated 10 minutes at 37°C, and the labeling reaction was stopped by adding a volume of FBS equal to the volume of cell suspension for 2 minutes at RT. The cells were washed and resuspended in TCM.

Untransduced PBMCs and CAR T cells (effector cells) were washed and suspended at 5 × 10⁶ cells/mL in TCM. The cytotoxicity of cMLVs conjugated and unconjugated CAR T cells was compared to the cytotoxicity of untransduced PBMCs, which was used as negative control.

The cultures were set up in triplicates in a sterile 96 well plate round bottom (Corning) at T cell:target cell (i.e., effector:target, E:T) ratios of 20:1, 10:1, 5:1 and 1:1. Each culture contains 50,000 SKOV3 (non-target) cells and 50,000 SKOV3-CD19⁺ (target) cells. After mixing, the cultures were spun down at 1000rpm for 30 seconds to pack the cells before incubating at 37°C for 4 hours. Immediately after the incubation, 7AAD (7-amino-actinomycin D) (BD Pharminogen) was added as recommended by the manufacturer. The fluorescence was analyzed by flow cytometry. Cell cytotoxicity was calculated as [CFSE⁺7AAD⁺ cells / (CFSE⁺7AAD⁻ + CFSE⁺7AAD⁺)] cells.

### Transmigration assay

T-cell transmigration assays were performed in 24 mm diameter 3 µm pore size Transwell plates (Costar). cMLVs conjugated and unconjugated CAR T cells (0.5 ×10⁶ /well) were plated on the upper wells and TCM was added to the lower wells. The T-cell chemoattractant CXCL-9 (0.1mg/ml, Preprotech) was added to the lower wells. After incubation at 37°C for 6 hours, T cells that have migrated into the lower chamber were counted.

### In vivo xenograft experiments of prevention study

SKOV3.CD19 tumors were implanted into NSG mice, as described above. After tumors were established, mice were randomly assigned to each treatment group. Tumor growth was measured using calipers and calculated using the formula (width² × length)/2. Three mice from each group were sacrificed on day two and day 14 post-treatment. The tumor and spleen from each mouse were harvested for further *ex vivo* analysis.

Corresponding to Figure 5G, a total of 3 ×10⁶ SKOV3-CD19⁺ cells in PBS solution were injected into the flanks of NOD/scid/IL2rγ-/- (NSG) mice. After tumors were established (100-150mm³), the mice were randomly assigned and received following treatments: (i) saline or PBS, (ii) CAR T cell (iii) CAR T cell conjugated to empty cMLVs and (iv) CAR T cell conjugated to SCH58261-loaded cMLVs. The mice in all of the CAR T cell treatment groups, mice were infused with 5 × 10⁶ CAR T cells (50% transduction). The tumor was monitored and measured using calipers and the tumor volume was calculated using the formula (width² × length)/2. Five mice from each group were sacrificed on day 2 and day 14 post adoptive CAR T cell transfer. The tumor, spleen and blood from each mouse were harvested and processed. The tumor and spleen were microdissected and passed through 70µm nylon meshes. Red blood cells in the spleen and blood samples were lysed. A total of 0.5 × 10⁶ cells from single cell suspensions were placed in round bottom 96 well plates (Corning) and stained with anti-human CD45, CD3, CD4 and CD8 antibodies to assess the infiltration amount of adoptively transferred CAR T cells.

### In vivo xenograft experiments of rescue study

SKOV3.CD19 tumors were implanted into NSG mice, as described above. After tumors were established, all the mice were injected with 3 × 10⁶ CART.tEGFR cells. Ten days after initial adoptive CAR-T cell transfer, the mice were randomly assigned to receive the following treatments: (1) PBS; (2) CAR-T cells; (3) CAR-T cells conjugated to empty cMLVs (CART.cMLV); (4) a mix of CAR-T cells and cMLV(SCH) (CART+cMLV(SCH)); and (5) CAR-T cells conjugated to SCH-loaded cMLVs (CART.cMLV(SCH)). Each mouse was injected with 2.5 × 10⁶ CAR-positive T cells. For mice treated with unconjugated cMLVs, 10⁹ drug-loaded cMLVs were co-infused with CAR-T cells. Forty-eight hours after the second adoptive T cell transfer, the mice were sacrificed. The spleen and tumor were harvested for ex vivo assays.

Corresponding to Figures 10A and 10B, a total of 3 ×10⁶ SKOV3-CD19⁺ cells in PBS solution were injected into the flanks of NOD/scid/IL2rγ-/- (NSG) mice. After tumors were established (100-150mm³), all the mice were injected with 20 × 10⁶ CART cells. Ten days post initial adoptive CART cell transfer, the mice were randomly assigned and received following treatments: (i) saline or PBS, (ii) CAR T cell (iii) CAR T cell conjugated to empty cMLVs and (iv) CAR T cell conjugated to SCH58261-loaded cMLVs. The mice in all of the CAR T cell treatment groups, mice were infused with 5 × 10⁶ CAR T cells (50% transduction). 48-hours post second adoptive T cell transfer, the mice were sacrificed. The spleen and tumor were harvested ex vivo assays. To assess the degree of T cell infiltration, the tumors and the spleens were microdissected and passed through 70µM nylon meshes. A total of 0.5 × 10⁶ cells from single cell suspensions were placed in round bottom 96 well plates (Corning) and stained with anti-human CD45, CD3, CD4 and CD8 antibodies to assess the infiltration amount of adoptively transferred CAR T cells.

### Ex vivo TIL analysis

Three analyses were performed: (1) anti-CD3/anti-CD28-induced intracellular IFN-γ cytokine staining, (2) phospho-CREB and (3) Ki-67 expression in CAR-T cells. For intracellular IFN-γ staining, a total of 0.5 × 106 cells were stimulated with human CD3/CD28 antibodies and 10 ng/mL Brefeldin A. The culture was incubated for 6 hours at 37°C in 96-well round bottom plates. Fluorophore-conjugated human CD3, CD45, CD4 and CD8 antibodies were used for immunostaining. Cytofix/Cytoperm solution (BD Bioscience) was used to permeabilize cell membrane and perform intracellular staining according to the manufacturer's instruction.

For intracellular phospho-CREB staining, cells were fixed with 4% paraformaldehyde (PFA), followed by permeabilization in methanol for 30 minutes on ice. Cells were then stained with Alexa488-conjugated anti-human phospho-CREB for 30 minutes at 4°C. Flow cytometry analysis was carried out using the MACSQuant^{®} Instrument from Miltenyi Biotec (Auburn, CA).

For Ki-67 staining, cells stained with fluorophore-conjugated human CD3, CD45 and EGFR were fixed with 80% ethanol and incubated at -20°C for 48h. Cells were washed twice with staining buffer (PBS with 1% FBS, 0.09% NaN₃), centrifuged for 10 minutes at 200 ×g and resuspended to a concentration of 10⁷ cells/mL. Cells were then stained with anti-Ki-67 antibody for 30 minutes at room temperature in the dark, washed twice with staining buffer, centrifuged at 200 ×g for 5 minutes, and analyzed by flow cytometry.

Corresponding to Figures 5L and 5M, the functional analyses were performed in two different assays (i) CD3 and CD28 induced IFNg expression by intracellular cytokine staining (ii) phospho CREB expression in TILs. A total of 0.5 x 10⁶ cells from single cell suspensions were placed in round bottom 96 well plates and stimulated with OKT3, anti-human CD28 antibodies and 10ng/mL Brefeldin. The culture is incubated at 37°C for 6 hours. After the incubation, the cells were labeled with anti-human CD3, CD45 and CD8 antibodies. Cells were then permeabilized in 100 µl of Cytofix/Cytoperm solution (BD Bioscience) at 4 °C for 20 minutes, washed with Perm/Wash buffer (BD Bioscience), and stained with PE-conjugated anti-human IFN-γ at 4 °C for 30 minutes. The flow cytometry analysis was carried out using the FACSort instrument from BD Biosciences.

### In vivo fluorescence confocal imaging and in vivo biodistribution study

For *in vivo* nanoparticle biodistribution study, a xenograft tumor model was used. To establish the tumor, SKOV3.CD19 cells in PBS solution were injected subcutaneously into the flanks of NOD/scid/IL2Rγ-/- (NSG) mice. DiD-labeled cMLVs (cMLV), CD19 CART cells (5 × 10⁶) mixed with DiD-labeled cMLVs (CART+cMLV), CD19 CAR-T cells (5 × 10⁶) surface-conjugated with DiD-labeled cMLVs (CART.cMLV), or PBS were intravenously injected into the tumor-bearing mice. After 24 and 48 hours, indicated tissues were removed, weighed, and macerated with scissors. DiD-specific tissue fluorescence (Abs 644 nm, Em 665 nm) was quantified for each organ using the Xenogen IVIS spectrum imaging system by the USC Imaging Core scientists blinded to the groups, and the percentage of injected dose per gram of tissue (%ID/g) was calculated.

Corresponding to figure 3C, CD19⁺CAR T cells (10 x 10⁶) surface-conjugated with DiD labeled nanoparticles were intravenously injected into NOD/scid/IL2rγ-/~ (NSG) mice bearing SKOV3 CD19+ tumors. After 48 hours indicated tissues were removed, weighed, and macerated with scissors. We quantified specific DiD tissue fluorescence for each organ using the Fluorescence Optical imaging system and calculated the fluorescence signal to background ratio as final readout.

For in vivo confocal imaging, Fluorescence images were acquired on a Yokogawa spinning-disk confocal scanner system (Solamere Technology Group, Salt Lake City, UT) using a Nikon eclipse Ti-E microscope equipped with a 60 × /1.49 Apo TIRF oil objective and a Cascade II: 512 EMCCD camera (Photometries, Tucson, AZ, USA). An AOTF (acousto-optical tunable filter) controlled laser-merge system (Solamere Technology Group Inc.) was used to provide illumination power at each of the following laser lines: 491 nm, 561 nm, and 640 nm solid state lasers (50 mW for each laser). To label liposomal particles, DiD lipophilic dyes were added to the lipid mixture in chloroform at a ratio of 0.01 : 1 (DiD : lipids), and the organic solvent in the lipid mixture was evaporated under argon gas to incorporate DiD dyes into a lipid bilayer of vesicles.

### Quantification of accumulated nanoparticles at tumor sites

SKOV3.CD19 tumors were implanted into NSG mice, as described above, and CFSE-labeled CAR-T cells and DiD-labeled cMLVs were injected into tumor-bearing mice. At the indicated times, tumors were excised, fixed, frozen, cryo-sectioned, and mounted onto glass slides. Fluorescence of CFSE-labeled CAR-T cells and DiD-labeled cMLVs was visualized using a Zeiss 700 Confocal Laser Scanning Microscope (Inverted) (Carl Zeiss, Germany). Quantification analysis was performed using Zeiss Zen microscope software.

Tumors were excised, fixed, frozen, cryo-sectioned, and mounted onto glass slides.

### Intratumoral PTX concentration measurements ex vivo

Using high performance liquid chromatography (HPLC), the PTX concentration in the frozen tumor tissues was quantified as previously detailed (35). Briefly, thawed *tumor* tissues were homogenized in ethyl acetate, with a known concentration of control drug added to each sample as an internal standard. The samples were centrifuged and the organic layer was transferred to a clean tube. The organic layer was evaporated under a stream of argon and rehydrated in diluted acetonitrile. After running the samples on HPLC, the peak heights were analyzed to determine intratumoral SCH concentration.

### Statistics

The differences between two groups were determined with Student's t test. The differences among three or more groups were determined with a one-way analysis of variance (ANOVA).

### In vitro drug encapsulation and release profiles

The amount of SCH encapsulated in the cMLV(SCH) was determined by C-18 RP-HPLC chromatography (Backman). The cMLV(SCH) suspension was diluted by adding water and acetonitrile to a total volume of 0.5 ml. Extraction of SCH was accomplished by adding 5 ml of tert-butyl methyl ether and mixing the sample by votex for 1 min. The mixtures were then centrifuged and the organic layer was transferred into a glass tube and evaporated to dryness under argon. Buffer A (95% water, 5% acetonitrile) was used to rehydrate the dried organic layer. To determine the SCH concentration, 1ml of the solution was injected into a C18 column, and SCH was detected at 392 nm (flow rate 1ml/min). The release kinetics of SCH from cMLVs were investigated by removing the releasing media from cMLVs incubated in 10% fetal bovine serum (FBS)-containing media at 37°C and replacing it with fresh media daily. The removed media was quantified for SCH fluorescence (by HPLC) daily. To obtain the release kinetics of SCH from cMLVs before and after cell conjugation, cMLV(SCH) and CART cMLV(SCH) were incubated in 10% FBS-containing media at 37 °C and were spun down and resuspended with fresh media daily. SCH was quantified from the harvested media every day by HPLC.

### Example 2

### Stable nanoparticle attachment to cell surfaces

To improve the efficacy of CAR-engineered T cell therapy, we used CAR-T cells as chaperones to carry nanoparticles loaded with SCH-58261, a drug that can inhibit an immune-suppressive mechanism in the TME. To express CARs on T cells, activated human PBMCs were transduced with a lentiviral vector to deliver anti-CD19 CAR consisting of CD28 and CD3ζ intracellular signaling domains. FACS analysis of surface CAR expression showed 50% transduction efficiency **(****FIG. 9****).**

Synthesized cMLV nanoparticles were stably coupled to the reduced thiol groups present on the cell surface via the thiol-reactive maleimide headgroups present on the lipid bilayer surface. According to previous reports, high levels of free thiols were detected on the surfaces of T cells, B cells and hematopoietic stem cells (HSCs). The conjugation is performed in two steps. First, the CAR-T cells and cMLVs with maleimide-functionalized lipids were coincubated to permit coupling to free thiols on the cell surface. After the initial coupling reaction, the conjugated cells underwent *in situ* PEGylation to quench residual reactive groups. **(****FIG. 1A****).** To determine the maximum numbers of particles that could be conjugated per T cell, we performed a serial dilution of the *nanoparticles* for conjugation at different ratios (5000 to 1, 1000 to 1, 500 to 1, 250 to 1 and 100 to 1). At a ratio of 1000:1, the conjugation of cMLVs reached a saturation point that resulted in an average of 287±49 surface-bound nanoparticles per cell **(****FIG. 1B** and **Table 1).**

The average conjugation efficiency of the nanoparticles on the T cell population was 55.9% **(****FIGs. 1C and 1D****)**. Moreover, single-cell imaging and three-dimensional reconstruction of CART.cMLVs demonstrated that the *nanoparticles* were distributed in several clusters on the cell surface **(****FIG. 1E****)**.

**Table 1: Number of conjugated nanoparticles (cMLVs) per T cell at different conjugation ratios.**

| **Conjugation Ratio** | **5000 to 1** | **1000 to 1** | **500 to 1** | **250 to 1** | **100 to 1** |
|---|---|---|---|---|---|
| **Number of cMLVs per cell** | 315 ± 48 | 287 ± 49 | 132 ± 27 | 7 ± 0 | 0 ± 0 |

### CAR-T cells conjugated with nanoparticles maintain T effector functions

We next sought to test whether surface-bound cMLVs could impact key cellular functions of CAR-T cells, such as cell cytokine secretion, cytotoxicity, and migration. CART cells with and without cMLV conjugation were co-cultured with either SKOV3.CD19 or K562.CD19 target cells for 4 hours. CART and CART.cMLV stimulated with SKOV3.CD19 target cells induced 17.05±0.07% and 19.15±1.63% IFN-γ+ T cell populations, respectively, indicating that both CART and CART.cMLV were able to secrete IFN-γ with similar efficiency **(****FIGs. 2A and 2B****).** When cMLVs were labeled with DiD dye, IFN-γ was secreted from both cells with and without surface-conjugated cMLVs **(****FIG. 10****).** Moreover, surface conjugation of cMLVs did not reduce CAR-T cell cytotoxicity against SKOV3.CD19 **(****FIG. 2D****)** or K562.CD19 cells **(****FIG. 2C****).** Lastly, we assessed CAR-T cell transmigration capabilities in vitro. Comparable percentages of conjugated and unconjugated cells migrated to the lower chamber of the transwell co-culture system, indicating that CART.cMLV cells maintain their transmigration capabilities **(****FIGs. 2E****, 2F).** Thus, the cell surface conjugation of cMLVs does not hinder recognition of target cells, IFN-γ secretion, cell cytotoxicity, or migration.

### Conjugation to CAR-T cells increases tumor localization and systemic circulation of cMLVs

To determine whether conjugation of cMLVs to CAR T cells could improve the accumulation of *nanoparticles* to the tumor site, we performed a biodistribution assay by synthesizing cMLVs containing DiD dye. DiD-labeled cMLVs alone (cMLV(DiD)), mixed with CAR-T cells (CART+cMLV(DiD)), or conjugated to CAR-T cells (CART.cMLV(DiD)) were intravenously injected into NSG mice bearing SKOV3.CD19 tumors, and DiD-tagged cMLV accumulation was monitored in different organs. At 24 hours, significantly higher cMLV accumulation was detected from CART.cMLV(DiD), compared to cMLV and CART + cML V groups, in the tumor (p<0.001), spleen (p<0.001), lymph node (p<0.01), and lung (p<0.01). No significant difference in cMLV accumulation was noted between cMLV(DiD) and CART+cMLV(DiD) groups in any tissues. Additionally, no significant difference in DiD signal was detected at 24 h in circulating blood in any group **(****FIG. 3A****).** At 48 hours, CART.cMLV(DiD) demonstrated higher cMLV accumulation in the blood (p<0.05), tumor (p<0.05), spleen (p<0.05), and lung (p<0.05) compared to both cMLV(DiD) and CART-cMLYYDiD) groups **(****FIG. 3B****).** Notably, CAR-T cell conjugation to cMLVs resulted in significantly lower cMLV accumulation in the liver compared to both cMLV(DiD) and CART+cMLV(DiD) groups at 24 (p<0.05) and 48 (p<0.001) hours.

The liposomes were either conjugated to CAR T cells prior to infusion or infused as free liposomes. After 48h, the mice were sacrificed and organs -i.e. tumor, liver, spleen, blood, kidney, lung, heart and lymph node, were isolated for optical microscopy. The results showed that there is insignificant homing difference between free liposome and conjugated liposome in every organ except the tumor, where liposomes conjugated to CAR T cells showed significantly higher homing compared to free liposomes. **(****Fig. 3C****)** This confirms our hypothesis that conjugation of cMLVs to CAR T cells would enhance the liposomes' *tumor* localization.

### Surface-conjugated cMLVs colocalize with CAR-T cells inside the tumor mass

We next evaluated the tumor infiltration properties of carrier CAR-T cells by *confocal* imaging of histological SKOV3.CD19 tumor sections that had been treated with cMLV-conjugated, or unconjugated, fluorescently labeled CAR-T cells. Representative *confocal* images demonstrate that the surface-conjugation of cMLVs does not impede intratumoral CAR-T cell migration. Both CART.cMLV and CART+cMLV had comparable infiltration of T cells **(****FIGs. 4A**, 4B). The density of CAR-T cells in the CART.cMLV- and CART+cMLV-treated *tumor* was 0.26±0.1 cells/mm² and 0.35± 0.2 cells/mm², respectively. However, the colocalization of CAR-T cells and cMLVs was only observed inside tumors treated with CART.cMLV. The percentage of colocalization was 78.57±26.7% in the CART.cMLV-treated tumors compared to no detection in CART+cMLV-treated tumors **(****FIGs. 4C**, 4D). These results indicate that cMLV conjugation to the CAR-T cells is able to increase the amount of cMLVs delivered to the tumor without impeding the migration of T cells.

### CAR-T cells conjugated with nanoparticles encapsulated with A2aR antagonist shows improved antitumor responses in vivo

To test whether the pharmacological inhibition of A2a receptor (A2aR) would prevent CAR-T cell hypofunction in the adenosine-rich TME, we monitored the *tumor* growth and T cell infiltration *in vivo.* SKOV3.CD19 tumor-bearing mice were assigned into six different groups as shown in **FIG. 5A****.** Animals in all treatment groups showed tumor progression. CART, CART.cMLV, CART+cMLV(SCH) and CART.cMLV(SCH) treatment groups showed statistically significant tumor growth control, from day 2 until day 17 post ACT, when compared to the PBS treatment group **(****FIG. 5B** and **Table 2).** Compared to other treatment groups, CART.cMLV(SCH) treatment demonstrated the most distinguished tumor growth inhibition resulting in significantly smaller tumors for all days measured post ACT. **(****FIG. 5B****)** Consequently, CART.cMLV(SCH) treatment markedly improved the survival of the mice compared to CART (p<0.0001, log-rank test), CART.cMLV (p<0.0001, log-rank test), and CART+cMLV(SCH) (p = 0.0008, log-rank test) treatment groups. CART, CART.cMLV, CART+cMLV(SCH) and CART cMLV(SCH) had a median survival of 22, 22, 24 and 36 days after treatment, respectively. **(****FIG. 5C****)** Both PBS and cMLV treatment had median survival of 22 days. Only CART.cMLV(SCH) treatment significantly improved the median survival compared to PBS and cMLV groups (p=0.0003 for PBS and p=0.0002 for cMLV, log-rank test).

**Table 2. Statistical analysis of the tumor growth curve at each time point compared to the PBS control.**

| **Days post ACT** | **PBS** | **cMLV(SCH)** | **CART** | **CART.cMLV** | **CART** | **CAKT.CMLV(SCH)** |
|---|---|---|---|---|---|---|
| 2 | | ns | ∗∗∗∗ | ∗∗∗∗ | ∗∗∗∗ | ∗∗∗∗ |
| 7 | | ns | ∗∗∗∗ | ∗∗∗∗ | ∗∗∗∗ | ∗∗∗∗ |
| 11 | | ns | ∗∗∗ | ∗∗∗ | ∗∗∗ | ∗∗∗∗ |
| 14 | | ns | ∗∗∗∗ | ∗∗∗∗ | ∗∗∗∗ | ∗∗∗∗ |
| 17 | | ns | ∗∗ | ∗∗∗∗ | ∗∗∗ | ∗∗∗∗ |
| 20 | | ns | ∗ | ns | ∗∗ | ∗∗∗∗ |

To explore how SCH affected tumor-infiltrating T cells, we analyzed T cell engraftment and functionality. CD3⁺ and CD45⁺ T cell engraftment in the tumor was evaluated on day 2 post-treatment. CART.cMLV(SCH) had higher T cell engraftment (52.96 ± 15.5%) compared to CART (15.06 ± 1.2%, p = 0.0134), CART.cMLV (15.36 ±1.9%, p = 0.0139) and CART + cMLV(SCH) (16.93 ±0.6%, p = 0.0157) treatment groups (FIG. 5D). Furthermore, we evaluated the functionality of tumor-infiltrating T cells that were exposed to the adenosine-rich immunosuppressive TME in vivo. CART cMLV(SCH) treatment group showed significantly higher intracellular IFN-γ expression (MFI = 744.24 ± 45.3) in CD45⁺ T cells compared to CART (MFI = 335.8 ± 91.2, p = 0.0023), CART.cMLV (MFI = 307.57 ± 53.6, p = 0.0004) and CART + cMLV(SCH) (611.52 ± 26.21, p = 0.0118) treatment groups. The free cMLV(SCH) treatment group, CART + cMLV(SCH) also resulted in an increased IFN-γ expression compared to CART (p = 0.0073) and CART.cMLV (p = 0.0009) treatments, although this level is less than observed in CART.cMLV(SCH) treatment (FIG. **5E****).**

To determine if the functional preservation of tumor-infiltrating T cells is, at least in part, the result of A2a receptor blockade, we tested the level of phosphorylated-CREB downstream of A2aR on isolated T cells. Our data showed that CD45⁺ T cells from the CART- and CART.cMLV-treated groups had significantly higher phosphorylated CREB compared to T cells harvested from the CART + cMLV(SCH) and CART.cMLV(SCH)-treated groups, indicating that SCH released from surface-engineered CAR-T cells could block A2a receptor signaling mediated by adenosine in TME. Notably, CART.cMLV(SCH) resulted in lower phosphorylated CREB compared to CART + cMLV(SCH) (p < 0.0001) **(****FIG. 5F****).** Overall, compared with free cMLV(SCH) treatment, conjugation of cMLV(SCH) to CAR-T cells significantly prolonged *tumor* growth inhibition, indicating higher efficiency in blocking the A2a receptor pathway and preventing CAR-T cell hypofunction.

Corresponding to Figure 5G, mice were inoculated with ovarian cancer cells (SKOV3-CD19⁺) that are engineered to over express the CD19 target. Once the tumor reaches 100-150mm³, 5 x 10⁶ CAR T cells -either unconjugated, or conjugated with cMLVs, or conjugated with cMLVs loaded with the small molecule inhibitor - were adoptively transferred via tail vein injection. The negative control tumor bearing mice were treated with PBS. The negative control did not show any control in tumor growth throughout the duration of the experiment. CART and CART-Emp group showed slight control within the first 7 days post adoptive transfer, although the growth progress as fast as the negative control group after day 7. Notably, the experimental group CART-SCH showed significant *tumor* growth control throughout the experiment. By day 14-post adoptive transfer, the tumor size of CART-SCH was 265±43 mm³. The average tumor size on day 14 of PBS, CART and CART-Emp were 859 (±245), 759 (±263) and 649 (±48) mm³, respectively. **(****Figure 5G****)**

Engraftment of T lymphocytes to the tumor and spleen were evaluated on day 2 and day 14 post adoptive T cell transfer. On day 2-post adoptive transfer, CD3⁺CD45⁺ T cells were detected in both the *tumor* and spleen. CART-SCH had significantly higher T cell engraftment (5.37% ±0.52) than both CART (2.98% ±0.46) and CART-Emp (3.26% ±0.51). **(****Figure 5H****)** T cell engraftment in the spleen, on the other hand, did not show any significant difference among the groups CART (0.03% ±0.03), CART-Emp (0.13% ±0.02) and CART-SCH (0.22% ±0.06). **(****Figure 5I****)** On day 14-post adoptive transfer, CD3⁺CD45⁺ T cells were still present in both the tumor and spleen. CART-SCH had significantly higher T cell engraftment (8.16% ±0.62) than both CART (5.0% ±0.45) and CART-Emp (5.26% ±0.39). **(****Figure 5J****)** By day 14-post adoptive transfer, there was significantly higher T cell engraftment in the spleen of mice treated with CART-SCH (10.35% ±1.6) compared to CART (5.29% ±0.75) and CART-Emp (5.27% ±0.32). **(****Figure 5K****)**

To evaluate the functionality of T cells once they have infiltrated to the tumor, we performed *ex vivo* TIL restimulation assay to evaluate the decrease of T cell effector function after being exposed to high adenosine immunosuppressive microenvironment. The effector function was compared to a positive control, which is the spleenocytes of a non-tumor bearing mice that received adoptive T cell transfer of 5 x 10⁶ cells per mouse. On day 2-post adoptive transfer, TILs of the experimental groups (PBS, CART, CART-Emp and CART-SCH) and spleenocytes of the positive control were stimulated with anti human CD3/CD28, and stained for intracellular IFNg cytokine secretion. Remarkably, TILs from all the experimental groups have already showed a decline in function compared to the positive control at 48h-post adoptive cell transfer. TILs from the group that received CART-SCH treatment have significantly higher intracellular IFNg secretion (MFI 3733 ±781) compared to CART (MFI 612 ±20) and CART-Emp (MFI 788 ±138) treated groups. (Figure 5L)

To determine if the preservation of TIL function is due to blockade of A2A receptor with the small molecule inhibitor SCH, we detected the intracellular phosphorylation level of cyclic AMP response element-binding protein (CREB). Binding of adenosine to A2AR activates G protein coupled receptors that leads to accumulation of cAMP, which is the upstream signaling element of cAMP-dependent protein kinase (PKA). In its basal state, PKA resides in the cytoplasm as an inactive heterotetramer of paired regulatory (R) and catalytic (C) subunits. Upregulation of cAMP releases the C subunits, which passively diffuse into the nuclease and phosphorylates CREB at serine residue 133. Therefore, T lymphocytes that encounter adenosine would have upregulated cAMP, which further induces CREB phosphorylation. On the contrary, successful blockade of A2A receptor with an antagonist would prevent intracellular accumulation of cAMP that results in lower CREB phosphorylation. Our data showed that TILs of CAR and CAR-Emp treated group had significantly higher phosphorylated CREB compared to TILs of the CAR-SCH treated group. (Figure **5M****)**

### CAR-T cells conjugated with A2aR antagonist-encapsulated nanoparticles are able to rescue hypofunctional tumor-residing T cells in vivo

Although tumor-infiltrated CAR-T cells can migrate into the tumor mass, they tend to gradually lose tumor killing and inflammatory cytokine secretion abilities after entering the adenosine-rich tumor microenvironment. We hypothesized that **hypofunctional** tumor-residing T cells could regain their effector functions upon the blocking of A2aR signaling with SCH. To demonstrate the potential of our conjugated system in this application, we established an in vivo model with **hypofunctional** tumor-residing CAR-T cells in the TME by an initial intravenous infusion of CD19 CAR-T cells that express a truncated epidermal growth factor receptor (tEGFR) to the tumor bearing mice (FIG. **11A****);** these CAR-T cells are designated as CART.tEGFR. The EGFR surface marker was used to trace the initial population of hypofunctional tumor-residing CAR-T cells, enabling us to distinguish them from the subsequent treatment dose of surface-engineered CAR-T cells lacking EGFR. Ten days after the initial CART.tEGFR cell transfer, the rescue treatment was infused to mice in five different groups **(****FIG. 6A****).**

Two days after the treatments, 5 out of 6 tumor-bearing mice that received CART.cMLV(SCH) treatment showed over 50% reduction in *tumor* size, with one mouse showing 44% reduction. The combination treatment group of CART+cMLV(SCH) showed more than 25% reduction in *tumor* size in 2 out of 6 mice. Tumor-bearing mice that received either CART or CART.cMLV had no significant reduction in tumor size, and the tumorbearing mice that received PBS treatment showed an overall increase in tumor size **(****FIGs. 6B****.** **11C** **and** **11D****).** Tumor-infiltrating T cells, including CAR-positive cells, were isolated from tumors for further ex vivo analysis. As shown in **FIG. 6D****,** CART.cMLV(SCH) treatment resulted in 10.79±0.3% total T cell population, which is significantly higher than all other treatment groups (CART, CART.cMLV, and CART+cMLV(SCH), p<0.001).

We further investigated the effect of these treatments on the initial hypofunctional CART.tEGFR cells. Tumors treated with CART, CART.cMLV, and CART+cMLV(SCH) had 25.65±2.8%, 25.35±0.5% and 28.90:1:3.1%) intratumoral CART.tEGFR, respectively, while CART.cMLV(SCH)-treated tumors had 63.08-1-5.8% CART.tEGFR cells, significantly higher than all other groups (p<0.001) (FIGs. 6C and 6E). The proliferation of CAR-T cells was assessed by the expression of Ki-67. CART.cMLV(SCH) showed significantly higher Ki-67 expression level (MFI = 3442.4:1:272.3) compared to other treatment groups: CART (MFI=1066.1±253.5, p=0.0004), CART.cMLV (MFI=1162.5±129.5, p=0.0002) and CART+cMLV(SCH) (MFI-1044.32±-224.8, p=0.0003)) **(****FIG. 6F****)**.

Next, we evaluated the ability of this CART-chaperoned drug to restore inflammatory function of the **hypofunctional** CART.tEGFR population. The CART.cMLV(SCH) treatment group showed significantly higher IFN-γ secretion in CART.tEGFR cells than that of CART, CART.cMLV, or CART+cMLV(SCH) groups (p<0.001) **(****FIG. 6G****).** Evaluation of pCREB expression levels in CART.tEGFR cells showed that the CART.cMLV(SCH) treatment significantly reduced pCREB level in CART.Tegfr cell populations compared to the CART, CART.cMLV, and CART+cMLV(SCH) treatment groups (p<0.001) **(****FIG. 6H****).** Taken together, this collective evidence suggests that the surface-engineered CART system can effectively deliver a small-molecule inhibitor of A2Ar to TME, thereby rescuing **hypofunctional** tumor-residing T cells *in vivo.*

Corresponding to figure 8, in cases where there are already TILs present in the tumor microenvironment, we utilize CART cells conjugated with cMLV as a delivery system to release A2AR antagonists in the *tumor* microenvironment for the purposes of rescuing TIL hypofunction. (Scheme according to **Figure 8****)** As we have shown previously **(****Figures 5L and 5M****),** TILs have decreased functionality almost immediately after they enter into an adenosine rich tumor microenvironment. Although they are still engrafted inside the tumor, they have lost their cytotoxocity, proliferation ability and inflammatory cytokine secretion. By interfering adenosine-A2AR activation with small molecule inhibitors, while not wishing to be bound by any particular theory, we hypothesized that **hypofunctional** TILs would regain its effector functions such as cytotoxicity, proliferation and inflammatory cytokine secretion. To demonstrate the potential of the CART-cMLV conjugated system in this application, we inoculated NSG mice with SKOV3-CD19⁺ and treated all tumor-bearing mice with 15 x 10⁶ CAR T cells. The purpose of the initial adoptive transfer of CAR T cells is to establish **hypofunctional** TILs in the tumor microenvironment. Although functionality declines almost immediately after T cell infiltration into the tumor site, we allowed 10 days before we performed a *second* adoptive cell transfer with different treatments -i.e. PBS, CART, CART-Emp and CART-SCH (5 x10⁶ cells per mouse). 48h post treatment, mice were sacrificed for analysis of TIL effector functions. **(Figure 6I)**

cMLVs containing A2A receptor antagonist (SCH58261) to CAR T initial adoptively transferred CAR T cells was able to induce reduction of *tumor* size within 48 hours post treatment by promoting TIL proliferation and improving effector T cell functions. The initial adoptive CAR T cell transfer (on day 0 as shown in **Figure 6I****)** did not significantly control *tumor* growth compared to un-treated *tumor* bearing mice. **(****figure 6J****)** Notably, 48h after the second adoptive CAR T cell therapy, the tumor-bearing mice that received CART-SCH showed 1/2 fold reduction in the tumor size compared to before the treatment. **(****Figure 6J****)** Tumor bearing mice that received either CART or CART-Emp showed insignificant reduction in tumor size 48h post adoptive cell transfer, and the *tumor* bearing mice that received PBS in the second round of treatment showed an increase in tumor size. **(****Figure 6J****)** Overall, significant reduction of the *tumor* size was observed only in the group treated with CART-SCH in the second infusion.

We further investigated the effect of second cell infusion treatment on CD3⁺ CD45⁺ T lymphocyte population in the tumor. Cells isolated from the tumor tissue were stained *ex vivo* with anti-human CD3 and CD45 antibodies. Tumor of mice treated with CART-SCH showed 39.27% (±6.57) T cell engraftment in the tumor, which is double the T cell percentage found in the tumor of CART and CART-Emp treatment groups that are 19.04.% (±8.63) and 17.82% (±8.31), respectively. **(****Figure 6K****)** The group that received only PBS in the second round of treatment still has T cell engraftment (6.09% ±1.68), although the percentage is lower than the other groups that received a second round of CART infusion. **(****Figures 6K and 6M****)** Moreover, we determined the CD8 to CD4 ratio of T lymphocytes from each group and found that treatment with CART-SCH induces a high CD8/CD4 ratio (1.24 ±0.05) or a CD8 biased T cell population. On the contrary, PBS, CART and CART-Emp treatments resulted in a CD4 biased population with CD8/CD4 ratios 0.427 ±0.15, 0.56 ±0.17 and 0.49 ±0.08, respectively. **(****Figure 6L****)**

Next, the effector function of TILs was assessed in an *ex vivo* assay where T lymphocytes were restimulated with CD3/CD28 antibodies to induce inflammatory cytokine secretion and intracellular IFNγ expression was detected. Spleenocytes of tumor free mice that received CART cell infusion were used as positive control. CART-SCH treatment group showed significantly higher IFNg secretion compared to CART and CART-Emp, although TILs from every group showed declined in function in comparison to the positive control. **(****Figure 6N****)**

Evaluation of phosphorylated CREB in T lymphocytes was also performed to determine that the small molecule inhibitor is interfering with A2A receptor activity. TILs from the PBS treated group were used as negative control because these T lymphocytes show characteristics of hypofunction, where T cells have inhibited effector functions. These **hypofunctional** TlLs from the PBS group show high-phosphorylated CREB. Treatment with CART-SCH significantly reduced phosphorylated CREB levels compared to both CART treatment and the negative control groups. **(****Figure 6O****)** Use of cMLV-CART conjugation system to deliver small molecule inhibitors of the A2A receptor was effective in rescuing **hypofunctional** TILs *in vivo.* Interference with adenosine activation through A2A receptor recovers the T cell effector functions, such as tumor cell killing (cytotoxicity) and inflammatory cytokine secretion, which leads to *tumor* size reduction and T lymphocyte proliferation.

Adoptive immunotherapy for cancer has been of interest for an extensive period of time, although the experiments have showed rather variable success. The first observations that suggested that the immune system has antitumor effects was reported by William Coley, who detected regression of sarcoma following sever bacterial infections in the 1890s. Research was conducted to explore the possibilities and procedure of utilizing the patients' own immune system to counteract the disease, but success was unpredictable and sporadic. CAR specific T cells have been of exceptional interest for clinical development. The potential of this approach was demonstrated in clinical trials, where T cells expressing CAR were infused into adult and pediatric patients with B-cell malignancies, neuroblastoma, and sarcoma. The success of CAR T cell therapy is limited in blood borne cancers. In order to improve upon the previous therapeutic methods, one of the strategies was to systematically infuse adjuvants to sustain and enhance the functions of the adoptively infused CAR T cells.

The combination therapy with adjuvants posed multiple complications, such as the effectiveness of the adjuvant itself and toxicity that comes with high administrative dose that is necessary for the adjuvant to show any effect at all. IL-2 infusion, for example, is one of the widely studied methods used to enhance antitumor immunity. Each study attempted to find the suitable administration route, dosage amount and frequency of administration. Thompson et al. (1987) demonstrated that IL-2 treatment, both intravenous and subcutaneous administration, did not lead to *tumor* regression and actually reduced the number of PBMC count, post treatment. In addition to the ineffectiveness, side effects or toxicity was observed. Patients showed dose limiting symptoms such as fever, hypotension and flu-like symptoms.[29] Toxicity of systemic infusion is a major concern that researchers have been investigating to avoid or minimize.

The utilization of liposomes or other nanoparticles (e.g., polymeric nanoparticles) as a drug carrier has emerged as an attractive delivery method in cancer therapy during the past decade. The synthesis of liposomes has become a common practice in multiple drug encapsulation procedures, such as paclitaxel and doxorubicin that are highly hydrophobic. A major defect of liposomes is its instability in the presence of serum components that causes fast-burst release of chemotherapeutic drugs, which consequently limits its utility for the delivery of chemotherapeutic agents. In order to improve on the traditional liposome synthesis, Moon et al (2011) and Joo et al (2013) developed methods to synthesize crosslinked multilamelar liposome (cMLVs), which can lower systemic toxicities and enhance therapeutic efficacy. Moreover, to enhance nanoparticle delivery to specific tumor sites, Stephan et al (2010) demonstrated that conjugation of liposome nanoparticles to the surface of mouse splenocytes and hematopoetic stem cells (HSCs) is stable both *in vitro* and *in vivo,* without instigating immunogenicity nor reduces the effector T cell functions.

Conjugation of cMLV to CAR modified or *tumor* specific lymphocytes is an effective method to enhance tumor specific homing of these nanoparticles, and consequently, reduce toxicity prompted by systemic infusion of chemotherapeutic drugs and adjuvants. The cell-bound nanoparticle delivery system has been tested for several applications. First, it was utilized as a targeted cytokine support of antitumor T cells. The *nanoparticles* were loaded with IL-15 and IL-21 that promotes *in vivo* T cell proliferation. The system was able to release bioactive cytokines over a 7-day period, and was able to promote complete tumor clearance as a result. In the same study, Stephen et al (2010) also proceeded to test the system by encapsulating GSK-3β small molecule inhibitor as therapeutic cargo to enhance the repopulation of donor HSCs. A third study was performed by Huang et al (2015), nanoparticles were loaded with topoisomerase I poison SN38 and conjugated to mouse lymphocytes to treat lymphoma in the lymph nodes.

Herein we demonstrated the cargo drug, protein or peptide does not cause toxicity on the therapeutic cells. *In* various embodiments, we excluded the delivery of chemotherapeutic drugs that were commonly used by others to obliterate tumor and tissues in general.

Based on the procedure of nanoparticle-cell surface conjugation developed in Stephen et al (2010), we pioneered the utilization of CAR engineered human PBMCs as therapeutic cells that have nanoparticles conjugated to the surface. The *nanoparticles* encapsulate small molecule inhibitors of the A2a receptor, e.g., SCH 58261, which is highly hydrophobic and is generally unsuitable for systemic infusion. Our *in vitro* XTT assay showed that SCH 58261 does not have cytotoxic effects against either PBMCs or tumor cells (SKOV3-CD19+). Therefore, SCH 58261 is a suitable drug to illustrate the advantage of this T cell-nanoparticle cargo system for the purposes of enhancing CAR T cell therapy by co-delivering therapeutic cells and adjuvant in the same unit. Compare with other compositions or systems to block the A2aR pathway, such as genetically engineering CAR-T cells with the CRISPR/Cas system or receptor siRNA knock down, a major advantage of presently disclosed composition and method is the ability of the drug to affect endogenous T cells and circulating CAR-T cells, as well as the carrier CAR-T cells themselves.

*In vitro,* we demonstrated that cMLV nanoparticles could be stably conjugated to the CAR-T cell surface while maintaining its ability to release loaded drug in a sustained manner and did not disrupt CAR-T cell effector functions.

Nanoparticles were successfully conjugated on the surface of human PBMCs. Previous research by Stephan et al (2010) showed that 140 (±30) of ~200nm nanoparticles were able to stably conjugate to the surface of mouse spleenocytes that have an average diameter of 7.6µm. Activated PBMCs, on the other hand, are able to stably conjugate up to 280 (±30) on the surface at the nanoparticle to T cell conjugation reaction ratio of 1000:1, and does not increase as we increase the reaction ratio to 5000:1. This is attributed to the larger size of activated PBMC that has a mean diameter of 10µm. This amount of conjugation does not perturb effector T cell functions such as T cell migration, cytokine secretion and cell cytotoxicity. Each nanoparticle conjugated T cell is able to encapsulate 28ng of SCH 58261, according to our calculations, which half the amount would be release within the first 48h-post conjugation. This amount is higher than the amount of drug, GSK-3β inhibitor SN-38, encapsulated per nanoparticle conjugated mouse spleenocyte (~0.4µg) reported in Huang et al (2015). Despite high dosage, conjugation of drug-loaded nanoparticles did not show toxicity against T cells, nor did it reduce the T cell's effector functions.

Our biodistribution study further shows that CAR-T cells enhance the efficacy of therapeutic drugs by actively directing drug-loaded nanoparticles to the tumor site in vivo, an event driven by the ability of CAR-T cells to migrate into the tumor mass through tumor-associated chemokine attraction. Overall, CART.cMLVS(DiD) had the highest particle accumulation at the tumor site at both 24 and 48 hours, reemphasizing the importance of cell-mediated delivery. Moreover, both cMLV(DiD) and CART+cMLV(DiD) resulted in significantly higher cMLV accumulation in the liver, which is where liposomal nanoparticles are typically cleared from the system by Kupffer and endothelial cells. while CART.cMLV(DiD) showed significantly lower cMLV accumulation in the liver, increased levels were observed in lymphoid tissues, such as the lymph node, spleen and lungs. These data provide evidence that CAR-T cell-bound nanoparticles may be retained in circulation for a longer period of time than free nanoparticles owing to reduced nanoparticle clearance by the liver.

Conjugation of cMLVs to CAR T cells allowed the nanoparticles to localize more efficiently at the tumor. We demonstrated that conjugated cMLVs had significantly higher accumulation in the tumor tissue compared to unconjugated nanoparticles. The common disadvantage of nanoparticles is their reliance on the enhanced permeability and retention effect (EPR) of blood vessels in the tumor microenvironment. These vessels are formed with defective endothelial cells with scattered apertures that trap nanoparticles in the tumor matrix that has no proper lymphatic drainage. Cancer treatment with nanoparticles, as a result, takes advantage of this property to deliver cancer chemotherapeutics to the tumor microenvironment. However, the limitation remains that the bulk of the tumor, where it is most hypoxic and unfavorable for immunosurveilance, often has low vascularization, which leads to poor disease prognosis as a result. Moreover, the EPR effect is heterogeneous and may be lacking completely in some tumors, which makes treatment with nanoparticles ineffective. CAR T cells, on the other hand, does not rely on the EPR effect because it has an innate migratory ability, and therefore it is able to deliver the conjugated cargo inside the tumor mass. Regarding other therapeutically relevant tissues (i.e. the liver, spleen, kidney, lung, heart, lymph node and blood), there is no significantly different infiltration amounts between free liposome and CART cell bounded liposome, although free liposome showed slightly higher homing everywhere else except the spleen and tumor. Moreover, our results did not show that conjugation of liposome reduces their infiltration to the liver, as previously reported in Stephen et al (2010).

In order to achieve maximal drug action on hypofuctional T cells within the TME, the drug-loaded nanoparticles must be able to reach the immune cells deep within the tumor mass. In this regard, the CART.cMLV drug delivery system promotes the colocalization of nanoparticles and CAR-T cells inside the tumor mass due to the innate mobility of T cells within the tumor to deliver drugs inside the TME (29,44). Confocal microscopic images showed that cMLVs from the CART.cMLV group were able to penetrate deep inside the tumor and colocalize with CAR-T cells. This maximum intratumoral localization of cMLV could be a major factor contributing to the higher potency of CART.cMLV(SCH) therapy.

Conjugation of cMLVs containing SCH 58261 to CAR T cells significantly improved tumor infiltration and their cytotoxicity *in vivo.* CART cells and CART cells conjugated to empty liposomes show limited cytotoxicity against tumor cells *in* vivo. Tumor growth was not suppressed after the mice received adoptive CART and CART-Emp cell transfer, although T cells were detected in the tumor microenvironment. Ex vivo functional assay showed that TILs of CART and CART-Emp cell groups have significantly lower inflammatory cytokine (IFNγ) secretion compared to CART-SCH. *Ex vivo* restimulation of TILs, at 48h post adoptive transfer, with anti-human CD3/CD28 showed that the effector function of CART cells is lost very soon after tumor infiltration. Previous research by Moon et al (2014) observed similar effects of the tumor microenvironment on T cell hypofunction. Cell cytotoxicity was reported to decline from day 5-post adoptive cell transfer and continue to decrease until day 39, despite the continuation of TIL proliferation. Here, we also observed that the percentage of TILs in the tumor increased in all the groups from day 2 to day 14-post adoptive T cell transfer, although the tumor size continued to increase. Further analysis of the signaling pathway downstream of the A2a receptor showed that CREB is highly phosphorylated in CART and CART-Emp treated group, and significantly less phosphorylated CREB was detected in CART-SCH treated tumors. This demonstrates that SCH 58261 inhibits adenosine signaling through A2a receptors on tumor infiltrated CAR T cells, which consequently displayed superior tumor growth suppression and proliferation *in vivo* compared to untreated CART cells.

The tumor-targeted CART.cMLV(SCH) therapeutic system was effective at preventing hypofunction of nanoparticle-conjugated CAR-T cells. Groups treated with CART.cMLV(SCH) demonstrated significant tumor growth suppression compared to groups without the conjugated drug. Prophylactic CART.cMLV(SCH) treatment showed high tumor engraftment of T cells with low CREB phosphorylation, indicating the mechanistic importance of A2aR blockade that leads to increased T cell proliferation. This is supported by our observation that CART.cMLV(SCH) had a higher percentage of tumorinfiltrated T cells and increased IFN-γ production compared to the other groups. It should be noted, however, that the maximal levels of IFN-γ *in* our most successful treatment group was still significantly lower than the control T cells isolated from tumor-free mice, suggesting that in addition to A2aR signaling, CAR-T cells could be exposed to other mechanisms for immunosuppression in this SKOV3 tumor model.

Further to the effect of cell bound drug carrier cMLVs in preventing therapeutic CART cell hypofunction, CART cells as simply chaperone cells to deliver cMLV encapsulated drug cargo to tumor residing T cells are herein studied, as "rescue" mission to recover hypofunctional TILs. For this application, our results showed that conjugation of cMLVs containing SCH 58261 to chaperone CAR T cells was able to stimulate proliferation and increase cytotoxicity of CAR T cells residing in the tumor. These tumor residing CAR T cells have been reported to be hypofunctional and have reduced cytotoxicity due to the immunosuppressive tumor microenvironment. In this circumstance, the drug cargo is aimed towards both chaperone CART cells and tumor residing T cells.

Unlike our preventative study where therapeutic CART-SCH was able to control tumor growth, chaperone CART-SCH was able to reduce tumor size dramatically within the first 48h post adoptive CART cell transfer. This significant reduction could be attributed to the blockade of A2a receptor, which consequently induced proliferation and increase effector functions of tumor residing T cells. The PBS control group that did not receive the second "rescue" infusion showed there is an average of 6% T cell residing in the tumor on day 10 post initial CART infusion. The second "rescue" infusion with either CART or CART-Emp slightly increased the TIL population to 19.2% and 18.3%, respectively. Notably, the "rescue" infusion with CART-SCH increased T cell population to reach 39.3% of total cells from harvested tumor. TILs from CART-SCH rescue treatment group showed CD8+ biased T cell population, which was not observed in either CART or CART-Emp treated group. CD8+ population was reported to be more sensitive to IFNγ levels in the tumor microenvironment. High IFNγ secretion significantly boosts CD8+ T cells proliferation, while having less effect on CD4+ T cell population. In agreement with these observations, harvested TILs from CART-SCH group expressed more IFNγ upon *ex vivo* restimulation with anti-human CD3/CD28. Lastly, further analysis of phosphorylated CREB levels showed that CART-SCH significantly reduced pCREB of harvested TILs.

This rescue treatment mirrors a clinical setting where patients have pre-existing TILs or have previously received CAR-T cell therapy. CART.cMLV(SCH) treatment resulted in significantly higher IFN-γ secretion in the initial hypofunctional CART.tEGFR cell population upon ex vivo restimulation compared to other treatment groups. In the CART.cMLV(SCH) treatment group, the CART.tEGFR population also showed lower phosphorylated CREB and significantly higher cell number compared to other groups, presumably due to the release of A2aR-mediated inhibition of T cell proliferation. We also confirmed that SKOV3.CD19 cells were not directly affected by SCH, indicating that SKOV3.CD19 tumor reduction was mainly achieved by the effect of SCH on the tumorinfiltrated CAR-T cells. Moreover, the immediate reduction of tumor burden after CART.cMLV(SCH) treatment is most likely caused by the recovery of cytotoxicity induced by the CART.tEGFR cell population. This is supported by the facts 1) CART.cMLV(SCH) treatment alone at the same dosage in our prophylactic study could only suppress, but not reduce, tumor growth; 2) tumor size reduction mediated by immediately infused T cells take 5-6 days, which is longer than 2 days we observed in this study.

The application of SCH 58261 to disrupt A2a receptor signaling manifests as a very promising method of reversing hypofunctional TILs *in vivo,* particularly in the cases of solid tumors with high CD39 and CD73 expression.

This delivery platform is highly flexible, and it can be applied to other drugs, cytokines, antibodies, or any combination thereof, the use of different therapeutic cells, such as tumor-specific T lymphocytes and hematopoietic stem cells (HSC) as targeted delivery vehicles, or other "chaperone" cells such as natural killer (NK) cells, may markedly increase the therapeutic efficacy of cytokines and a small-molecule inhibitor. Moreover, immune regulatory drugs could be delivered in combination with immune checkpoint blockade, such as anti-PD-1, to further promote antitumor immunity. For example, the composition may incorporate small molecule inhibitors of VFGF and EGFR in the nanoparticles that are bound to cell surfaces, and the composition is administered with αPD1 and αPD-L1.

Cell-mediated drug delivery by surface engineering of CAR-T cells with nanoparticles not only enables controlled drug effect on the carrier cells, but also allows active targeting to the tissue of interest. By using CAR-T cells as chaperones, we were able to efficiently localize nanoparticles in specific tissues favorable for T cell homing, including tumor, spleen, lungs and lymph nodes. This method of combining CAR-T cell immunotherapy with A2aR small molecule antagonists could also be applied to various types of cancers -such as breast, prostate, brain cancers and leukemia. These cancers have been reported to express CD73, which is associated with poor prognosis. Overall, this is a promising platform that can potentially improve the efficacy and specificity of solid tumor therapies.

## Claims

1. An engineered immune effector cell, comprising:
a. an immune effector cell; and
b. particles chemically bonded to the surface of the immune effector cell;
wherein the immune effector cell contains polynucleotides encoding one or more chimeric antigen receptors (CARs) or expresses CARs, and
wherein the particles encapsulate an inhibitor of adenosine A_{2A} receptor (A2AR).

2. The engineered immune effector cell of claim 1, wherein the immune effector cell comprises one or more of NK cell, T cell, B cell, tumor-specific T lymphocyte and hematopoietic stem cell, optionally wherein the immune effector cell is autologous.

3. The engineered immune effector cell of claim 1, wherein the A2AR inhibitor comprises SCH58261, caffeine, SYN115, FSPTP, ZM241385, PBS-509, ST1535, ST4206, Tozadenant, V81444, or Istradefylline, optionally wherein the A2A receptor inhibitor is SCH58261.

4. The engineered immune effector cell of claim 1, wherein the particles are nanoparticles or microparticles comprising liposomes, or are polymeric particles.

5. The engineered immune effector cell of claim 3, wherein the particles are crosslinked multilayer liposome (cMLV).

6. The engineered immune effector cell of claim 1, wherein the immune effector cell is a T cell, the particles are cMLV, and the inhibitor of A2AR is SCH58261, and/or wherein the antigen targeted by the CARs are any one or more of 4-1BB, 5T4, adenocarcinoma antigen, alpha-fetoprotein, BAFF, B-lymphoma cell, C242 antigen, CA-125, carbonic anhydrase 9 (CA-IX), C-MET, CCR4, CD152, CD19, CD20, CD200, CD22, CD221, CD23 (IgE receptor), CD28, CD30 (TNFRSF8), CD33, CD4, CD40, CD44 v6, CD51, CD52, CD56, CD74, CD80, CEA, CNTO888, CTLA-4, DR5, EGFR, EpCAM, CD3, FAP, fibronectin extra domain-B, folate receptor 1, GD2, GD3 ganglioside, glycoprotein 75, GPNMB, HER2/neu, HGF, human scatter factor receptor kinase, IGF-1 receptor, IGF-1, IgG1, L1-CAM, IL-13, IL-6, insulin-like growth factor I receptor, integrin α5β1, integrin αvβ3, MORAb-009, MS4A1, MUC1, mucin CanAg, N-glycolylneuraminic acid, NPC-1C, PDGF-R a, PDL192, phosphatidylserine, prostatic carcinoma cells, RANKL, RON, ROR1, SCH 900105, SDC1, SLAMF7, TAG-72, tenascin C, TGF beta 2, TGF-β, TRAIL-R1, TRAIL-R2, tumor antigen CTAA16.88, VEGF-A, VEGFR-1, VEGFR2, vimentin and combinations thereof.

7. The engineered immune effector cell according to claim 1, for use as a medicament.

8. Engineered immune effector cells for use in treating, inhibiting, or reducing the severity or likelihood of cancer in a subject in need thereof, wherein each engineered immune effector cell comprises:
a. an immune effector cell; and
b. particles chemically bonded to the surface of the immune effector cell;
wherein the immune effector cell contains polynucleotides encoding one or more chimeric antigen receptors (CARs) or expresses CARs, and
wherein the particles encapsulate an inhibitor of adenosine A_{2A} receptor (A2AR).

9. Engineered immune effector cells for use in treating, inhibiting, or reducing the severity or likelihood of a disease in a subject in need thereof, wherein each engineered immune effector cell comprises:
a. an immune effector cell; and
b. crosslinked multilayer liposomes (cMLVs) bonded to the surface of the immune effector cell;
wherein the immune effector cell contains polynucleotides encoding one or more CARs or expresses CARs, and
wherein the cMLVs encapsulate an inhibitor of adenosine A_{2A} receptor.

10. Engineered immune effector cells for use according to claim 9, wherein the immune effector cells comprise one or more of NK cells, T cells, B cells, tumor-specific T lymphocytes and hematopoietic stem cells, optionally wherein the immune effector cell is a T cell, the particles are cMLV, and the inhibitor of A2AR is SCH58261.

11. Engineered immune effector cells for use according to claim 9, wherein the A2A receptor inhibitor comprises SCH58261, caffeine, SYN115, FSPTP, ZM241385, PBS-509, ST1535, ST4206, Tozadenant, V81444, or Istradefylline, and/or wherein the A2A receptor inhibitor is SCH58261.

12. Engineered immune effector cells for use according to claim 9, wherein the disease is cancer.

13. Engineered immune effector cells for use according to claim 9, wherein the subject receives an existing therapy selected from the group consisting of chemotherapy, radiation therapy and combinations thereof, and/or wherein the existing therapy is administered sequentially or simultaneously with the engineered immune effector cells.

14. Engineered immune effector cells for use according to claim 9, wherein the antigens targeted by the CARs are any one or more of 4-1BB, 5T4, adenocarcinoma antigen, alpha-fetoprotein, BAFF, B-lymphoma cell, C242 antigen, CA-125, carbonic anhydrase 9 (CA-IX), C-MET, CCR4, CD152, CD19, CD20, CD200, CD22, CD221, CD23 (IgE receptor), CD28, CD30 (TNFRSF8), CD33, CD4, CD40, CD44 v6, CD51, CD52, CD56, CD74, CD80, CEA, CNTO888, CTLA-4, DR5, EGFR, EpCAM, CD3, FAP, fibronectin extra domain-B, folate receptor 1, GD2, GD3 ganglioside, glycoprotein 75, GPNMB, HER2/neu, HGF, human scatter factor receptor kinase, IGF-1 receptor, IGF-1, IgG1, L1-CAM, IL-13, IL-6, insulin-like growth factor I receptor, integrin α5β1, integrin αvβ3, MORAb-009, MS4A1, MUC1, mucin CanAg, N-glycolylneuraminic acid, NPC-1C, PDGF-R a, PDL192, phosphatidylserine, prostatic carcinoma cells, RANKL, RON, ROR1, SCH 900105, SDC1, SLAMF7, TAG-72, tenascin C, TGF beta 2, TGF-β, TRAIL-R1, TRAIL-R2, tumor antigen CTAA16.88, VEGF-A, VEGFR-1, VEGFR2, vimentin and combinations thereof.

15. Engineered immune effector cells for use according to claim 12, wherein the subject has pre-existing tumor infiltrating lymphocytes (TILs) or has previously received CAR-T cell therapy.

## Patentansprüche

1. Gentechnisch veränderte Immuneffektorzelle, umfassend:
a. eine Immuneffektorzelle; und
b. Partikel, die chemisch an die Oberfläche der Immuneffektorzelle gebunden sind;
wobei die Immuneffektorzelle Polynukleotide enthält, die einen oder mehrere chimäre Antigenrezeptoren (CARs) kodieren, oder CARs exprimiert, und
wobei die Partikel einen Inhibitor des Adenosin-A_{2A}-Rezeptors (A2AR) einkapseln.

2. Gentechnisch veränderte Immuneffektorzelle nach Anspruch 1, wobei die Immuneffektorzelle eine oder mehrere von NK-Zellen, T-Zellen, B-Zellen, tumorspezifischen T-Lymphozyten und hämatopoetischen Stammzellen umfasst, wobei die Immuneffektorzelle optional autolog ist.

3. Gentechnisch veränderte Immuneffektorzelle nach Anspruch 1, wobei der A2AR-Inhibitor SCH58261, Koffein, SYN115, FSPTP, ZM241385, PBS-509, ST1535, ST4206, Tozadenant, V81444 oder Istradefyllin umfasst, wobei der A2A-Rezeptor-Inhibitor optional SCH58261 ist.

4. Gentechnisch veränderte Immuneffektorzelle nach Anspruch 1, wobei die Partikel Nanopartikel oder Mikropartikel sind, die Liposomen umfassen, oder Polymerpartikel sind.

5. Gentechnisch veränderte Immuneffektorzelle nach Anspruch 3, wobei die Partikel vernetzte mehrschichtige Liposomen (crosslinked multilayer liposome - cMLV) sind.

6. Gentechnisch veränderte Immuneffektorzelle nach Anspruch 1, wobei die Immuneffektorzelle eine T-Zelle ist, die Partikel cMLV sind und der Inhibitor von A2AR SCH58261 ist und/oder wobei das Antigen, auf das die CARs abzielen, eines oder mehrere von 4-1BB, 5T4, Adenokarzinom-Antigen, Alphafetoprotein, BAFF, B-Lymphomzelle, C242-Antigen, CA-125, Carboanhydrase 9 (CA-IX), C-MET, CCR4, CD152, CD19, CD20, CD200, CD22, CD221, CD23 (IgE-Rezeptor), CD28, CD30 (TNFRSF8), CD33, CD4, CD40, CD44 v6, CD51, CD52, CD56, CD74, CD80, CEA, CNTO888, CTLA-4, DR5, EGFR, EpCAM, CD3, FAP, Fibronektin-Extradomäne B, Folatrezeptor 1, GD2, GD3-Gangliosid, Glykoprotein 75, GPNMB, HER2/neu, HGF, Human-Scatter-Factor-Rezeptorkinase, IGF-1-Rezeptor, IGF-1, IgG1, L1-CAM, IL-13 , IL-6, insulinähnlicher Wachstumsfaktor-I-Rezeptor, Integrin α5β1, Integrin avß3, MORAb-009, MS4A1, MUC1, Mucin CanAg, N-Glycolylneuraminsäure, NPC-1C, PDGF-R a, PDL192, Phosphatidylserin, Prostatakarzinomzellen, RANKL, RON, ROR1, SCH 900105, SDC1, SLAMF7, TAG-72, Tenascin C, TGF beta 2, TGF-β, TRAIL-R1, TRAIL-R2, Tumorantigen CTAA16.88, VEGF-A, VEGFR-1, VEGFR2, Vimentin und Kombinationen davon sind.

7. Gentechnisch veränderte Immuneffektorzelle nach Anspruch 1 zur Verwendung als Medikament.

8. Gentechnisch veränderte Immuneffektorzellen zur Verwendung beim Behandeln, Hemmen oder Reduzieren der Schwere oder Wahrscheinlichkeit von Krebs bei einem Subjekt, das dies benötigt, wobei jede gentechnisch veränderte Immuneffektorzelle Folgendes umfasst:
a. eine Immuneffektorzelle; und
b. Partikel, die chemisch an die Oberfläche der Immuneffektorzelle gebunden sind;
wobei die Immuneffektorzelle Polynukleotide enthält, die einen oder mehrere chimäre Antigenrezeptoren (CARs) kodieren, oder CARs exprimiert, und
wobei die Partikel einen Inhibitor des Adenosin-A_{2A}-Rezeptors (A2AR) einkapseln.

9. Gentechnisch veränderte Immuneffektorzellen zur Verwendung beim Behandeln, Hemmen oder Reduzieren der Schwere oder Wahrscheinlichkeit einer Erkrankung bei einem Subjekt, das dies benötigt, wobei jede gentechnisch veränderte Immuneffektorzelle Folgendes umfasst:
a. eine Immuneffektorzelle; und
b. vernetzte mehrschichtige Liposomen (cMLVs), die an die Oberfläche der Immuneffektorzelle gebunden sind;
wobei die Immuneffektorzelle Polynukleotide enthält, die einen oder mehrere CARs kodieren, oder CARs exprimiert, und
wobei die cMLVs einen Inhibitor des Adenosin-A_{2A}-Rezeptors einkapseln.

10. Gentechnisch veränderte Immuneffektorzellen zur Verwendung nach Anspruch 9, wobei die Immuneffektorzellen eine oder mehrere von NK-Zellen, T-Zellen, B-Zellen, tumorspezifischen T-Lymphozyten und hämatopoetischen Stammzellen umfassen, wobei optional die Immuneffektorzelle eine T-Zelle ist, die Partikel cMLV sind und der Inhibitor von A2AR SCH58261 ist.

11. Gentechnisch veränderte Immuneffektorzellen zur Verwendung nach Anspruch 9, wobei der A2A-Rezeptor-Inhibitor SCH58261, Koffein, SYN115, FSPTP, ZM241385, PBS-509, ST1535, ST4206, Tozadenant, V81444 oder Istradefyllin umfasst, und/oder wobei der A2A-Rezeptor-Inhibitor SCH58261 ist.

12. Gentechnisch veränderte Immuneffektorzellen zur Verwendung nach Anspruch 9, wobei die Erkrankung Krebs ist.

13. Gentechnisch veränderte Immuneffektorzellen zur Verwendung nach Anspruch 9, wobei das Subjekt eine bestehende Therapie erhält, ausgewählt aus der Gruppe, die aus Chemotherapie, Strahlentherapie und Kombinationen davon besteht, und/oder wobei die bestehende Therapie nacheinander oder gleichzeitig mit den gentechnisch veränderten Immuneffektorzellen verabreicht wird.

14. Gentechnisch veränderte Immuneffektorzellen zur Verwendung nach Anspruch 9, wobei die Antigene, auf die die CARs abzielen, eines oder mehrere von 4-1BB, 5T4, Adenokarzinom-Antigen, Alpha-Fetoprotein, BAFF, B-Lymphomzelle, C242-Antigen, CA-125, Carboanhydrase 9 (CA-IX), C-MET, CCR4, CD152, CD19, CD20, CD200, CD22, CD221, CD23 (IgE-Rezeptor), CD28, CD30 (TNFRSF8), CD33, CD4, CD40, CD44 v6, CD51, CD52, CD56, CD74, CD80, CEA, CNTO888, CTLA-4, DR5, EGFR, EpCAM, CD3, FAP, Fibronectin-Extradomäne B, Folatrezeptor 1, GD2, GD3-Gangliosid, Glykoprotein 75, GPNMB, HER2/neu, HGF, Human-Scatter-Factor-Rezeptorkinase, IGF-1-Rezeptor, IGF-1, IgG1, L1-CAM, IL-13, IL-6, insulinähnlicher Wachstumsfaktor-I-Rezeptor, Integrin α5β1, Integrin avß3, MORAb-009, MS4A1, MUC1, Mucin CanAg, N-Glycolylneuraminsäure, NPC-1C, PDGF-R a, PDL192, Phosphatidylserin, Prostatakarzinomzellen, RANKL, RON, ROR1, SCH 900105, SDC1, SLAMF7, TAG-72, Tenascin C, TGF beta 2, TGF-β, TRAIL-R1, TRAIL-R2, Tumorantigen CTAA16.88, VEGF-A, VEGFR-1, VEGFR2, Vimentin und Kombinationen davon sind.

15. Gentechnisch veränderte Immuneffektorzellen zur Verwendung nach Anspruch 12, wobei das Subjekt vorbestehende tumorinfiltrierende Lymphozyten (TILs) hat oder zuvor eine CAR-T-Zelltherapie erhalten hat.

## Revendications

1. Cellule effectrice immunitaire conçue, comprenant :
a. une cellule effectrice immunitaire ; et
b. des particules liées chimiquement à la surface de la cellule immunitaire effectrice ;
dans laquelle la cellule effectrice immunitaire contient des polynucléotides codant pour un ou plusieurs récepteurs antigéniques chimériques (CAR) ou exprime les CAR, et
dans laquelle les particules encapsulent un inhibiteur du récepteur de l'adénosine A_{2A} (A2AR).

2. Cellule effectrice immunitaire conçue selon la revendication 1, dans laquelle la cellule effectrice immunitaire comprend un ou plusieurs parmi la cellule NK, le lymphocyte T, le lymphocyte B, le lymphocyte T spécifique de la tumeur et la cellule souche hématopoïétique, dans laquelle éventuellement la cellule effectrice immunitaire est autologue.

3. Cellule effectrice immunitaire conçue selon la revendication 1, dans laquelle l'inhibiteur de l'A2AR comprend SCH58261, la caféine, SYN115, FSPTP, ZM241385, PBS-509, ST1535, ST4206, le Tozadénant, V81444, ou l'Istradéfylline, éventuellement dans laquelle l'inhibiteur du récepteur A2A est SCH58261.

4. Cellule effectrice immunitaire conçue selon la revendication 1, dans laquelle les particules sont des nanoparticules ou des microparticules comprenant des liposomes, ou sont des particules de polymères.

5. Cellule effectrice immunitaire conçue selon la revendication 3, dans laquelle les particules sont des liposomes multicouches réticulés (cMLV).

6. Cellule effectrice immunitaire conçue selon la revendication 1, dans laquelle la cellule effectrice immunitaire est un lymphocyte T, les particules sont des cMLV, et l'inhibiteur de l'A2AR est SCH58261, et/ou dans laquelle l'antigène ciblé par les CAR sont l'un ou plusieurs parmi 4-1BB, 5T4, l'antigène de l'adénocarcinome, l'alpha-fœtoprotéine, BAFF, la cellule de lymphome B, l'antigène C242, CA-125, l'anhydrase carbonique 9 (AC-IX), C-MET, CCR4, CD152, CD19, CD20, CD200, CD22, CD221, CD23 (récepteur de l'IgE), CD28, CD30 (TNFRSF8), CD33, CD4, CD40, CD44 v6, CD51, CD52, CD56, CD74, CD80, CEA, CNTO888, CTLA-4, DR5, EGFR, EpCAM, CD3, FAP, l'extra-domaine B de la fibronectine, le récepteur de folate 1, GD2, le ganglioside GD3, la glycoprotéine 75, GPNMB, HER2/neu, HGF, la kinase du récepteur du facteur de diffusion humain, le récepteur de l'IGF-1, IGF-1, IgG1, L1-CAM, IL-13, IL-6, le récepteur I du facteur de croissance analogue à l'insuline, l'intégrine α5β1, l'intégrine αvβ3, MORAb-009, MS4A1, MUC1, la mucine CanAg, l'acide n-glycolylneuraminique, NPC-1C, PDGF-R a, PDL192, la phosphatidylsérine, les cellules de carcinome prostatique, RANKL, RON, ROR1, SCH 900105, SDC1, SLAMF7, TAG-72, la ténascine C, TGF-bêta 2, TGF-β, TRAIL-R1, TRAIL-R2, l'antigène tumoral CTAA16.88, VEGF-A, VEGFR-1, VEGFR2, la vimentine et les combinaisons de ceux-ci.

7. Cellule effectrice immunitaire conçue selon la revendication 1, destinée à être utilisée en tant que médicament.

8. Cellules effectrices immunitaires conçues pour une utilisation dans le traitement, l'inhibition ou la réduction de la gravité ou de la probabilité d'un cancer chez un sujet qui en a besoin, dans lesquelles chaque cellule effectrice immunitaire conçue comprend :
a. une cellule effectrice immunitaire ; et
b. des particules liées chimiquement à la surface de la cellule effectrice immunitaire ;
dans lesquelles la cellule effectrice immunitaire contient des polynucléotides codant pour un ou plusieurs récepteurs antigéniques chimériques (CAR) ou exprime les CAR, et
dans lesquelles les particules encapsulent un inhibiteur du récepteur de l'adénosine A_{2A} (A2AR).

9. Cellules effectrices immunitaires conçues pour une utilisation dans le traitement, l'inhibition ou la réduction de la gravité ou la probabilité d'une maladie chez un sujet qui en a besoin, dans lesquelles chaque cellule effectrice immunitaire conçue comprend :
a. une cellule effectrice immunitaire ; et
b. des liposomes multicouches réticulés (cMLV) liés à la surface de la cellule effectrice immunitaire ;
dans lesquelles la cellule effectrice immunitaire contient des polynucléotides codant pour un ou plusieurs CAR ou exprime les CAR, et
dans lesquelles les cMLV encapsulent un inhibiteur du récepteur de l'adénosine A_{2A}.

10. Cellules effectrices immunitaires conçues pour une utilisation selon la revendication 9, dans lesquelles les cellules effectrices immunitaires comprennent un ou plusieurs parmi les cellules NK, les lymphocytes T, les lymphocytes B, les lymphocytes T spécifiques de la tumeur et les cellules souches hématopoïétiques, éventuellement dans lesquelles la cellule effectrice immunitaire est un lymphocyte T, les particules sont des cMLV, et l'inhibiteur de l'A2AR est SCH58261.

11. Cellules effectrices immunitaires conçues pour une utilisation selon la revendication 9, dans lesquelles l'inhibiteur du récepteur A2A comprend SCH58261, la caféine, SYN115, FSPTP, ZM241385, PBS-509, ST1535, ST4206, le Tozadénant, V81444, ou l'Istradéfylline, et/ou dans lesquelles l'inhibiteur du récepteur A2A est SCH58261.

12. Cellules effectrices immunitaires conçues pour une utilisation selon la revendication 9, dans lesquelles la maladie est le cancer.

13. Cellules effectrices immunitaires conçues pour une utilisation selon la revendication 9, dans lesquelles le sujet reçoit une thérapie existante choisie dans le groupe constitué par la chimiothérapie, la radiothérapie et les combinaisons de celles-ci, et/ou dans lesquelles la thérapie existante est administrée séquentiellement ou simultanément avec des cellules effectrices immunitaires conçues.

14. Cellules effectrices immunitaires conçues pour une utilisation selon la revendication 9, dans lesquelles les antigènes ciblés par les CAR sont l'un ou plusieurs parmi 4-1BB, 5T4, l'antigène de l'adénocarcinome, l'alpha-fœtoprotéine, BAFF, la cellule de lymphome B, l'antigène C242, CA-125, l'anhydrase carbonique 9 (AC-IX), C-MET, CCR4, CD152, CD19, CD20, CD200, CD22, CD221, CD23 (récepteur de l'IgE), CD28, CD30 (TNFRSF8), CD33, CD4, CD40, CD44 v6, CD51, CD52, CD56, CD74, CD80, CEA, CNTO888, CTLA-4, DR5, EGFR, EpCAM, CD3, FAP, l'extra-domaine B de la fibronectine, le récepteur de folate 1, GD2, le ganglioside GD3, la glycoprotéine 75, GPNMB, HER2/neu, HGF, la kinase du récepteur du facteur de diffusion humain, le récepteur de l'IGF-1, IGF-1, IgG1, L1-CAM, IL-13, IL-6, le récepteur I du facteur de croissance analogue à l'insuline, l'intégrine α5β1, l'intégrine αvβ3, MORAb-009, MS4A1, MUC1, la mucine CanAg, l'acide n-glycolylneuraminique, NPC-1C, PDGF-R a, PDL192, la phosphatidylsérine, les cellules de carcinome prostatique, RANKL, RON, ROR1, SCH 900105, SDC1, SLAMF7, TAG-72, la ténascine C, TGF-bêta 2, TGF-β, TRAIL-R1, TRAIL-R2, l'antigène tumoral CTAA16.88, VEGF-A, VEGFR-1, VEGFR2, la vimentine et les combinaisons de ceux-ci.

15. Cellules effectrices immunitaires conçues pour une utilisation selon la revendication 12, dans lesquelles le sujet a des lymphocytes infiltrant une tumeur préexistante (TIL) ou a déjà reçu une thérapie cellulaire CAR-T.
